# EUROPEAN PATENT SPECIFICATION

(11) **EP 1 223 952 B1**
(45) Date of publication and mention of the grant of the patent: **16.04.2008**
(21) Application number: 00968760.9
(22) Date of filing: 07.10.2000
(51) Int. Cl.: A61K 35/76, C12N 15/861, C12N 9/12, A61P 35/00, A61K 48/00

(54) **METHODS FOR TREATMENT OF SOLID TUMORS AND METASTASIS BY GENE THERAPY**
METHODEN ZUR BEHANDLUNG VON FESTEN TUMOREN UND METASTASEN MIT GENTHERAPIE
METHODES DE TRAITEMENT DE TUMEURS SOLIDES ET DE METASTASES PAR THERAPIE GENIQUE

(30) Priority: 07.10.1999 US 158068 P
(43) Date of publication of application: 24.07.2002
(73) Proprietor: Aguilar-Cordova, Carlos, Estuardo, Newton, MA (US)
(72) Inventor: Aguilar-Cordova, Carlos, Estuardo, Newton, MA (US)
(74) Representative: Tollett, Ian
(86) International application number: PCT/US2000/027547
(87) International publication number: WO 2001/024684

(56) References cited:
- US-A- 5 601 818
- US-A- 5 747 469
- HERMAN J R ET AL: "IN SITU GENE THERAPY FOR ADENOCARCINOMA OF THE PROSTATE: A PHASE I CLINICAL TRIAL" HUMAN GENE THERAPY, XX, XX, vol. 10, 1 May 1999 (1999-05-01), pages 1239-1249, XP000983940 ISSN: 1043-0342
- KIM JAE HO ET AL: "Selective radiosensitization of 9L glioma in the brain transduced with double suicide fusion gene" CANCER JOURNAL FROM SCIENTIFIC AMERICAN, vol. 4, no. 6, November 1998 (1998-11), pages 364-369, XP008036191 ISSN: 1081-4442
- ROGULSKI K R ET AL: "Pronounced antitumor effects and tumor radiosensitization of double suicide gene therapy" CLINICAL CANCER RESEARCH 1997 UNITED STATES, vol. 3, no. 11, 1997, pages 2081-2088, XP008036203 ISSN: 1078-0432
- KIM JAE HO ET AL: "Selective enhancement by an antiviral agent of the radiation -induced cell killing of human glioma cells transduced with HSV-tk gene" CANCER RESEARCH, AMERICAN ASSOCIATION FOR CANCER RESEARCH, BALTIMORE, MD, US, vol. 54, no. 23, 1994, pages 6053-6056, XP002168821 ISSN: 0008-5472
- BLACKBURN ROBERT V ET AL: "Adenoviral transduction of a cytosine deaminase/thymidine kinase fusion gene into prostate carcinoma cells enhances prodrug and radiation sensitivity" INTERNATIONAL JOURNAL OF CANCER, vol. 82, no. 2, 19 July 1999 (1999-07-19), pages 293-297, XP002300054 ISSN: 0020-7136
- NISHIHARA EIJUN ET AL: "Retrovirus-mediated herpes simplex virus thymidine kinase gene transduction renders human thyroid carcinoma cell lines sensitive to ganciclovir and radiation in vitro and in vivo" ENDOCRINOLOGY, vol. 138, no. 11, 1997, pages 4577-4583, XP002298505 ISSN: 0013-7227
- FREYTAG S O ET AL: "A novel three-pronged approach to kill cancer cells selectively: concomitant viral, double suicide gene and radiotherapy" HUMAN GENE THERAPY, XX, XX, vol. 9, 10 June 1998 (1998-06-10), pages 1323-1333, XP002956490 ISSN: 1043-0342
- LOENING STEFAN A: "Gold seed implantation in prostate brachytherapy" SEMINARS IN SURGICAL ONCOLOGY, vol. 13, no. 6, 1997, pages 419-424, XP008036266 ISSN: 8756-0437
- DATABASE UNKNOWN [Online] ROGULSKI K.R. ET AL.: 'Glioma cells transduced with an escherichia coli CD/HSV-1 TK fusion gene exhibit enhanced metabolic suicide and radiosensitivity', XP002939333 Retrieved from DIALOG & HUMAN GENE THERAPY vol. 8, no. 1, 01 January 1997, pages 73 - 85
- TONG X. ET AL.: 'Adenovirus mediated thymidine kinase gene therapy may enhance sensitivity of ovarian cancer cells to chemotherapeutic agents' ANTICANCER RESEARCH vol. 18, no. 5A, September 1998 - October 1998, pages 3421 - 3426, XP002939334
- HANNA N.N. ET AL: 'Virally directed cytosine deaminase/5-fluorocytosine gene therapy enhances radiation response in human cancer xenografts' CANCER RESEARCH vol. 57, 01 October 1997, pages 4205 - 4209
- Summary of presentation at ASCO June 2004: Results of phase 2 clinical trial of ProstatakTM in prostate cancer
- SATOH T. ET AL: 'Enhanced systemic T-cell activation after in situ gene therapy with radiotherapy in prostate cancer patients' INT. J. RADIATION ONCOLOGY BIOL. PHYS. vol. 59, no. 2, 2004, pages 562 - 571
- VLACHAKI M.T. ET AL: 'Enhanced therapeutic effect of multiple injections of HSV-TK+GCV gene therapy in combination with ionizing radiation in a mouse mammary tumor model' INT. J. RADIATION ONCOLOGY BIOL. PHYS. vol. 51, no. 4, 2001, pages 1008 - 1017
- AGUILAR L.K. ET AL: 'PSA nadir and 24 month biopsy interim analysis of AdV-tk/Valacyclovir gene therapy in combination with radiotherapy vs radiotherapy alone for prostate cancer' JOURNAL OF CLINICAL ONCOLOGY vol. 22, no. 14S, 15 July 2004, page 4571
- RAINOV N.G.: 'A phase III clinical evaluation of Herpes Simplex virus type 1 thymidine kinase and ganciclovir gene therapy as an adjuvant to surgical resection and radiation in adults with previously untreated glioblastoma multiforme' HUMAN GENE THERAPY vol. 11, 20 November 2000, pages 2389 - 2401

## Description

### TECHNICAL FIELD

The present invention relates generally to cancer therapy, and more specifically to methods of inhibiting the growth of solid tumors and metastasis utilizing vector constructs, prodrug activating technology, immunostimulatory constructs and their use in ways that are compatible with current clinical standards of care.

### BACKGROUND OF THE INVENTION

### PREVALENCE OF CANCER

Cancer affects millions of people. Cancer is the second leading cause of death and accounts for one-fifth of the total mortality in the United States. There are many different types, primary and metastatic and the following are some of the predicted morbidities and mortalities in the USA (S.H.Landis, T. Murray, S. Bolden and P.A.Wingo, CA Cancer J. Clin. 49:8-31, 1999).

In 1999 there will be an estimated 1,221,800 new cancer cases. It is estimated that there will be 623,800 new male cases, and 598,000 new female cases. There will be 270,000 genital tumors (uterine cervix, uterine corpus, ovary, vulva, vagina, prostate, testis, and others) 226,000 digestive tumors (esophagus, stomach, small intestine, colon, rectum, anus, pancreas, liver, and others), 180,000 newly diagnosed breast cancers. Other tumors that comprise the rest of newly diagnosed cancers include, urinary system (86,500), lymphoma (64,000) respiratory system (187,600), brain and other nervous system (17,000), skin (54,000). Nonsolid tumors (leukemia) only account for 30,000 of the newly diagnosed tumors.

It is estimated that there will be 560, 000 deaths in the USA due to cancer in 1999 (290,000 male, and 270,000 female). In the female population it is estimated that there will be 61,000 from the digestive system, 69,000 from the respiratory system (mostly lung) 43,000 from breast cancer, and other solid tumor sites comprising the rest of the 270,000. Nonsolid tumors will account for approximately 9,700 cancer deaths. In the male it is estimated that there will be 70,000 deaths from digestive tumors, 95,000 from the respiratory system (mostly lung), 37,000 from prostate cancer, and other tumor sites comprising the rest of the 290,000 cases. Only 12,000 of the estimated cancer deaths are from nonsolid tumors.

A majority of the deaths from cancer are due to the systemic metastatic effects of the disease. Metastatic disease is the growth of tumor cells outside of their site of origin. It is not uncommon for some cancer patients to have undetected metastasis at the time of original diagnosis, these are sometime referred to as micrometastasis.

### CURRENT THERAPIES

Cancer is characterized by the uncontrolled division of a population of cells. This uncontrolled division typically leads to the formation of a tumor, which may subsequently metastasize to other sites.

The current therapies for cancer can be divided into four categories: surgery, radiation, chemotherapy and immunotherapy.

Surgical oncology can be further divided into three distinct areas: curative, debulking, and palliative. The principal goal of surgery is to completely remove the tumor and obtain "clear" surgical margins. It is often not possible to completely resect the tumor because of location or because of local invasion into surrounding normal tissues. Examples of these are tumors that have invaded locally into nerve roots, muscle or bone. Surgery is the only therapy required for tumors that are diagnosed at an early stage that have not metastasized via the blood stream or the lymphatics, or invaded sensitive or inaccessible tissues. Although even in those situations additional therapy may be indicated. For example, women who undergo lumpectomies often receive a course of radiation after their surgery to prevent recurrence. A thorough staging evaluation is required before the patient is deemed probably curable by surgery alone. This staging work-up is performed to show that there is no spread of disease. Even after this many patients recur after what is deemed complete tumor resection. Surgery is also used as a debulking procedure where by the tumor has been or will be further inhibited by chemotherapy, radiotherapy or both chemotherapy and radiation therapy. The reason for performing radiation and chemotherapy first is because the tumor may initially be too large to remove and must be shrunk before clear surgical margins can be obtained. The other role that surgery plays is palliation (relief of symptoms). In this sense it is not used with curative intent but is only offered to relieve symptoms. A case of palliative oncologic surgery is the patient that has disease compressing the spinal cord and the tumor is removed to relieve the symptoms of pain and neurological deficit. In short surgery treats cancer by removing it.

Radiation therapy is used in approximately 50% of the patients that have cancer. Radiation therapy is used with curative, adjuvant or palliative intent. There are certain cancers that can be cured by radiation therapy alone. The group of patients judged to be able to benefit from radiation monotherapy are those that have been fully staged and are found to have local or local or loco-regional disease. Analogous to the way the surgeon uses the scalpel, the radiation oncologist uses the radiation beam to destroy cancer cells by causing physical damage, such as double strand breaks in the DNA of the cell. The radiation can be generated in high doses, low doses, short term or long term. The sources can be radioactive seeds, radioactive source probes or external beams such as the high energy x-rays that are produced by a machine called a linear accelerator. Cancers that, in some situations, be can be cured by radiation alone include prostate cancer, head and neck cancer, cervical cancer, brain tumors and other cancers. However, as in all other cancer therapy modalities, there are many patients that recur.

Most often radiation therapy is used in an adjuvant setting. The radiation is given either before or after surgery or chemotherapy. Examples of these include the patient with breast cancer that is given radiation after lumpectomy; or the patient that has head and neck cancer that has been surgically removed and is at high risk for local recurrence; or a patient that does not have clear surgical margins. This same type of interaction can occur with chemotherapy. For example, when a tumor has been shrunk by chemotherapy and radiation is given to eradicate remaining tumor cells not destroyed by the chemotherapy, or when radiation and chemotherapy are mutually sensitizing, such as with 5-fluorouracil.

The other way that radiation therapy is used is in the palliative setting where it can relieve symptoms of bone pain or neurological symptoms related to the brain or spinal cord compression.

Chemotherapy works by interfering with different phases of the cell cycle or intercalating with the DNA of the cancer cell. As with other modalities, it is used with curative intent, in the adjuvant setting, concomitantly with radiation, or it can be used palliatively. The regimen of chemotherapy that is used is dependent on the disease site and pathological subtype. Chemotherapy is given systemically and acts on cancer cells throughout the body. This systemic effect is unlike radiation and surgery that only destroy the cancer cells at the site of therapy. Chemotherapeutic agents are most often given as multi-drug regimens in order to take advantage of the different mechanisms of action of each drug and to avoid single drug-resistant mutant-cell proliferation.

Chemotherapy has shown good curative outcomes in patients with testicular tumors and lymphomas. More commonly, chemotherapy is given in the adjuvant setting in combination with surgery, radiation, or both. For example, chemotherapy is given prior to radiation in patients with non-small cell lung cancer, bladder cancer, head and neck cancer and Hodgkin's lymphoma. It is given concomitantly with radiation for patients small cell lung cancer, anal carcinoma, most gastro-intestinal malignancies, (stomach, rectum, esophagous, pancreas). It is given after surgery for bladder cancer, pancreatic cancer, stomach cancer, breast cancer and other solid tumors. It is used as a palliative agent for the patients that have pain or neurological symptoms.

### LIMITATIONS OF CURRENT THERAPIES

The standard therapies above have significant limitations. None are 100% curative and all have associated toxicities. Surgery and radiation have the limitation that they can only treat locally or loco-regional disease. There is also a dose limit to the amount of radiation that can be given before there is too much damage to normal neighboring structures. Similarly, there is a limit to how much a surgeon may remove in order to get a clear surgical margin. Removal of too much normal tissue can cause excessive morbidity to the patient. Chemotherapy affects all tissues of the body since it is given systemically. Different chemo-therapeutic agents affect different organs differently. The most commonly affected organ is the bone marrow, where the drop in blood counts limits the dose of chemotherapy that can be given to the patient. But there can also be significant toxicity to kidneys, liver, gastrointestinal tract, and other tissues.

Primary solid tumors are generally treated by surgical resection. However, the majority of patients who have solid tumors also possess micrometastases beyond the primary tumor site. If treated with surgery alone, approximately 70% of these patients will experience recurrence of the cancer. Therefore, in addition to surgery, many cancers are also treated with a combination of therapies involving cytotoxic chemotherapeutic drugs (e.g., vincristine, vinblastine, cisplatin, methotrexate, 5-FU, etc.) and/or radiation therapy. One difficulty with this approach, however, is that radiotherapeutic and chemotherapeutic agents are toxic to normal tissues, and often create life-threatening side effects. In addition, these approaches often have extremely high failure/remission rates (up to 90% depending upon the type of cancer).

### DEVELOPING THERAPIES

In addition to surgery, chemo- and radiation therapies, many have attempted to bolster or augment an individual's own immune system in order to eliminate the cancer cells. Several immunotherapies have utilized bacterial or viral components as adjuvants, in order to stimulate the immune system to destroy the tumor cells. Examples of such components include BCG, endotoxin, mixed bacterial vaccines, interferons (alpha, beta, and gamma), interferon inducers (e.g., Brucella abortus, and various viruses), and thymic factors (e.g., thymosin fraction 5, and thymosin alpha-*1) (see generally* "Principles of Cancer Biotherapy," Oldham (ed.), Raven Press, New York, 1987). Such agents have generally been useful as adjuvants and as nonspecific stimulants in animal tumor models, but have not yet proved to be generally effective in humans.

Lymphokines have also been utilized in the treatment of cancer. Briefly, lymphokines are secreted by a variety of cells, and generally have an effect on specific cells in the generation of an immune response. Examples of lymphokines include TNF-alpha, interferons, interleukins (e.g. IL-1, -2, -3, -4, and -12 as well as colony stimulating factors such as G-CSF, GM-CSF, and M-CSF). Recently, one group has utilized IL-2 to stimulate peripheral blood cells in order to expand and produce large quantities of cells that are cytotoxic to tumor cells (Rosenberg et al., N. Engl. J Med. 313:1485-1492, 1985). Another group of agents being studied are called chemokines. These are agents that attract immune cells to a site and thus may stimulate an immune response. An example of these is Rantes.

Others have suggested the use of antibody-mediated anti-cancer therapies. Briefly, antibodies may be developed which recognize certain cell surface antigens that are either unique, or more prevalent on cancer cells compared to normal cells. These antibodies, or "magic bullets," may be utilized either alone or conjugated with a toxin in order to specifically target and kill tumor cells (Dillman, "Antibody Therapy," Principles of Cancer Biotherapy, Oldham (ed.), Raven Press, Ltd., New York, 1987). For example, Ball et al. (Blood 62:1203-1210, 1983) treated several patients with acute myelogenous leukemia with one or more of several monoclonal antibodies specific for the leukemia, resulting in a marked decrease in circulating leukemia cells during treatment. Similarly, others have utilized toxin-conjugated antibodies therapeutically to treat a variety of tumors, including, for example, melanomas, colorectal carcinomas, prostate carcinomas, breast carcinomas, and lung carcinomas *(see* Dillman, *supra).* One difficulty however, is that most monoclonal antibodies are of murine origin, and thus hypersensitivity against the murine antibody may limit its efficacy, particularly after repeated therapies. Common side effects include fever, sweats and chills, skin rashes, arthritis, and nerve palsies. Recently humanized monoclonals have been tested and at least one has been approved, namely "Herceptin/Trastuzumab", a monclonal antibody that recognizes the her2-neu marker on some tumor cells. This has been approved for treatment of metastatic breast cancer (Goldenberg, Clin. Therapy 21: 309-318, 1999) but its impact on the treatment of breast cancer remains to be determined.

### GENE THERAPY (GENE DELIVERY VEHICLES)

Gene therapy vectors can be classified as two main types - viral and non-viral. Both types are reviewed in detail in Methods in Human Gene Therapy, T. Freidmann Ed. Cold Spring Harbor Press, 1999, which is hereby incorporated by reference. The most commonly used viral systems are retroviral vectors and adenoviral vectors, in part for historical reasons and in part because they have been relatively straightforward to make in clinically useful quantities. These vectors have both been used extensively in the clinic, and some clinical trials have also been conducted using Adeno-associated viral vectors, rhabdoviruses, herpes viral vectors and vectors based on vaccinia virus or poxviruses. These viruses have various strengths and weaknesses, but are all relatively efficient in delivering genes to target tissues. Limitations include difficulties in making sufficient quantities for some vectors, inability to accurately target the gene delivery in vivo, toxic or immunological side effects of viral gene products. However it should be noted that even with the relatively efficient viral vectors it is not reasonable at present to expect that a gene can be delivered to every sick cell, and so therapy need to be accomplished by means that are compatible with this issue.

Non viral systems include naked DNA, DNA formulated in lipososomes and DNA formulated with polycation condensing agents or hybrid systems. These systems are more amenable to building in rational regulated steps to accomplish a long in vivo half-life, delivery to the target cell/tissue, entry into the cytoplasm and nucleus and then subsequent expression. Although there are possible solutions to each of these issues, they have not yet been efficiently combined, and efficiency of gene transfer in vivo remains an issue at this time. So for these systems also, it is not reasonable to expect to be able to deliver a gene to every cell, for example in a tumor.

Therefore, in cancer therapies using gene delivery vehicles, it is necessary to use mechanisms that allow some kind of amplification of the gene delivery events. These may include stimulation of the immune system, various forms of bystander effects, spread of apoptosis, antiangiogenic effects, pro-coagulant effects, replication competent viral vectors or other mechanisms.

### CANCER GENE THERAPY METHODS

Because of the generally dismal outlook for many cancer patients and the plateau in improvements with conventional therapies such as those described above, there has been considerable interest in using novel modalities such as gene therapy for treatment of cancer (see F. Farzaneh, U. Trefzer, W. Sterry and P. Walden: "Gene therapy of Cancer" Immunology Today 19: 294-296, 1998). These include various paradigms such as: introduction of genes for various cytokines and chemokines in order to generally stimulate immunity against the tumor and its presumed tumor associated antigens (TAAs e. g. Addison et al. Gene Ther. 5: 1400-1409 1998); administration of genes for specific TAAs to stimulate immunity against these antigens (e. g. Schlom & Hodge lmmunol Rev. 170: 73-84 1999); treatment with tumor suppressor or apoptosis inducing genes such as p53 (Eastham JA J. Urol. 164: 814-9 2000); treatment with genes the products of which can lead to suppression of angiogenesis or otherwise diminish blood-flow to tumor sites (e.g. Grischelli et al Proc Natl Acad Sci USA 95: 6367-6372 1998, WO 96/21416); treatment with genes the products of which will metabolize inactive compounds into active anti-tumor compounds (Connors, Gene Ther. 2: 702-709 1995, Deonarian et al. Gene Ther. 2: 235-244 1995); and combinations of these (e. g. Soler MN, Cancer Immunol Immunother. 48: 91-99 1999); various other modalities such as those designed to potentiate T cell anti-tumor responses. The vectors used include retroviral vectors, adenoviral vectors, naked and formulated DNA, herpes viral vectors, adeno-associated vectors, and many other kinds of vectors (see T. Friemann 1999 op. cit.). Animal experiments have shown the possibility of success with some of these modalities and vectors and many of these continue to have promise. However, as noted above, there are logistical difficulties in implementing trials and putting into use many of these therapies. This has been exacerbated because gene therapy approaches have often been seen as monotherapies or used alone in patients with end-stage disease. The introduction and use of gene therapy approaches would be simpler if it were understood what their interactions with standard of care therapies were and it were used as an adjuvant therapy in all stages of disease.

A number of animal tumor studies have been performed with adenoviral vector encoding the Herpes virus thymidine kinase (HSVTK, AdTk, AdV-tk, etc) (J. A. Eastham et al. Hum. Gene Ther. 7 515-523 1996; M. Y. Hurwitz et al. Human Gene Ther. 9: 1323-1333 1998, and references therein). In addition replicating adenoviral vectors encoding TK (O. Wildner etal. Gene Therapy 6: 57-62 1999) and the use of adenoviral vectors encoding prodrug activating genes (PDAG) with radiation therapy (Freytag et al Hum. Gene Ther. 9: 1323-1333 1998 and references therein) have been reported in animal models. However implementation of these therapies in the clinic face considerable issues as described in this specification. In addition clinical trial results with an adenoviral vector encoding TK in conjunction with gancyclovir has also been reported in patients suffering from prostate cancer with local recurrence after radiotherapy at stage T2 (J.R. Herman, Hum. Gene Ther. 10:1239-1249 1999). From this data it seems unlikely that Adeno-TK monotherapy will be very useful in treatment of solid tumors.

### PROBLEMS IN INTRODUCING NEW THERAPIES

Clinical trials conducted in an ethical and legal manner follow established rules of conduct and practice (e.g. see the US Code of Federal Regulations - Chpt. 21). Such guidelines are enforced and policed by patient advocacy groups, the National Institute of Health, the ethical standards and advice of the physicians, by Institutional Review Boards set up to specifically review such issues in every trial conducted at the specific medical institution, and by the national drug approval agency (in the USA this is the Food and Drug Administration, FDA). Trials are therefore conducted in a manner that does not deprive a patient of the most appropriate treatment that would normally be used on that patient (the clinical standard of care). Therefore it is difficult to determine the efficacy of new therapies, especially in cancer, as stand-alone treatments for the disease. The only context where such therapies can be introduced clinically is when all other therapies have been tried, and no therapy is left that is expected to have a clinical benefit. Such patients usually have advanced disease with short life expectancy. This makes the testing of such new therapies very difficult and unlikely to yield positive results, given the advanced stage of disease. An alternative to this approach is to test candidate therapies that can be used in the context of current standard of care and simply be given in addition to current therapy. This demands that the new therapy at least does not negativeiy interact with current therapy and also vice versa. Ideally the new therapy would be expected to show collaborative effects with the current clinical treatment. In addition it is difficult and expensive during trials and also for new therapies if extra medical care such as infusions requiring hospitalization or extensive home care are required. Therefore any new therapy that adds a minimum of extra hospital care or extensive home care will be more acceptable to patients, physicians and those institutions paying for healthcare. This in turn will make these therapies more likely to go through to approval by healthcare authorities and be used by treating physicians.

Blackburn et al. (1999) Int. J Cancer 82, 293-7 discloses adenoviral transduction of a cytosine deaminase/thymidine kinase fusion gene into prostate carcinoma cells. Although it was concluded that prodrug and radiation sensitivity were enhanced, these authors suggest that *in vivo* the strong immune responses elicited by adenoviral infection could be problematic.

Kim et al. (1994) Cancer Research 54, 6053-6 discloses an *in vitro* study of radiation-induced killing of human glioma cells transduced with a retroviral vector encoding thymidine kinase. The authors suggest that at the drug concentrations that could be achieved in humans, anti-herpes agents are not effective radiation sensitizers and that genes other than thymidine kinase should be investigated.

Hanna et al. (1997) Cancer Research 57, 4205-9 discloses the use of cytosine deaminase and 5-fluorocytosine gene therapy to enhance radiation response in human cancer xenografts. The authors beiieved that this approach would not be suitable for human cancers because the therapeutic genes could not be delivered efficiently enough *in vivo.* Furthermore, the immunogenicity of adenoviruses is indicated as counteracting any anti-tumour effect.

According to an aspect of the present invention, there is provided use of a replication-deficient adenoviral gene delivery vector as specified in claim 1.

This and other aspects of the invention will become evident upon references to the attached detailed description and drawings.

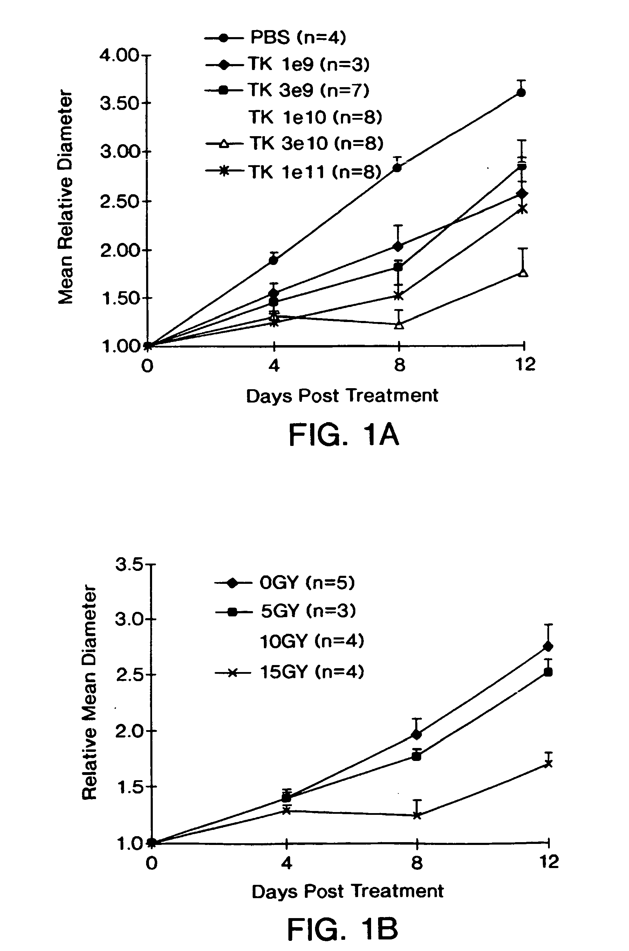

Figure 1 shows single therapy dose escalation analyses. Subcutaneous RM-1 tumors of 50-60 mm³ were used to determine effective but non-curative single therapy doses to be used in the combination therapy studies. A). Vector doses were analyzed by single AdV. RSV-tk intratumoral injections at doses ranging from 1x10⁹ to 1x10¹¹ v. p. per tumor in half log increments followed by six days of GCV at 20 mg/kg bid. B). Radiation doses were analyzed by single-fraction delivery of 5,10, and 15 Gy as described in Example 2.

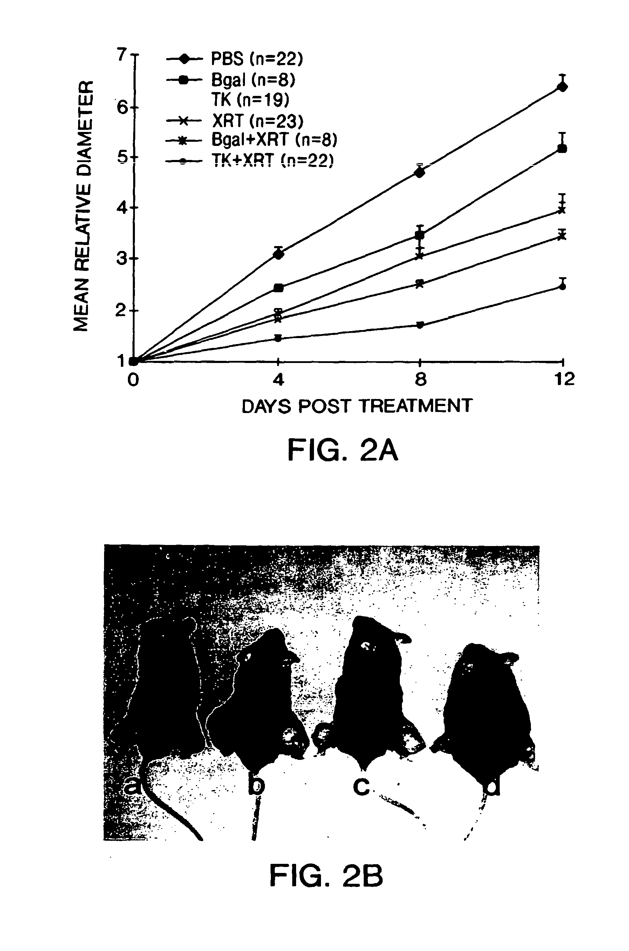

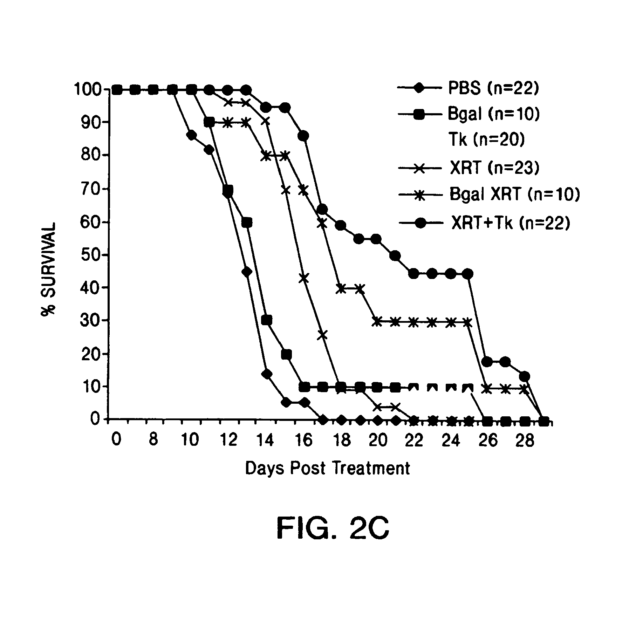

Figure 2 shows combination therapy effects on tumor growth in a mouse prostate tumor model. Tumors that received adenoviral vectors were injected with 3x10¹⁰ AdV-tk or AdV-β-gal v.p. Irradiated tumors received a single dose of 5 Gy. A). Relative tumor growth post treatment delivery of control, single therapy and combined therapy groups. B). C57BL/6 mice ten days after treatment with: a) AdV-b-gal+5Gy; b) AdV-tk only; c) 5 Gy only; and d) AdV-tk+5Gy. Tumor masses can be seen in both flanks of the animals. C) relative survival of the treated and control animals.

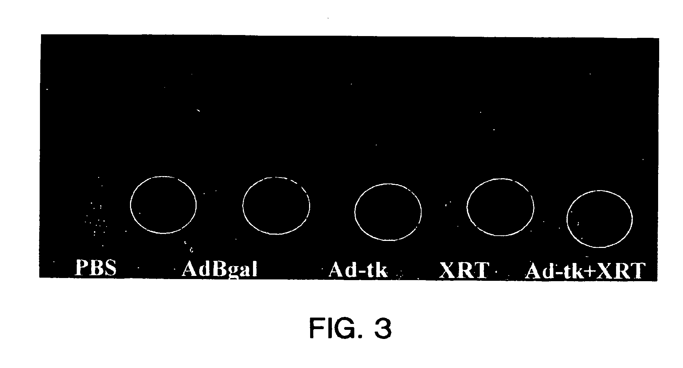

Figure 3 shows combination therapy effects on tumor growth in a mouse mammary tumor model. Tumors that received adenoviral vectors as described above. Balb/c mice ten days after treatment with AdV-b-gal+5Gy; AdV-tk only; 5 Gy only; and AdV-tk+5Gy as indicated. Tumor masses can be seen in the right flanks of the animals.

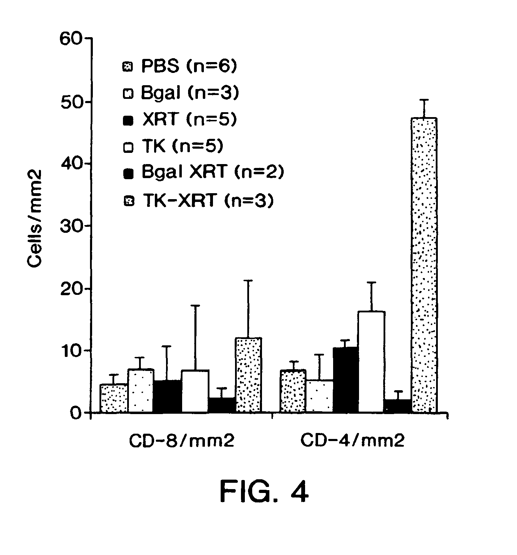

Figure 4 shows the immunoeffect of the treatment. Results of immune cell infiltrate of tumors after treatment with single modalities or combined therapy. Frozen sections of each tumor were stained with antibodies specific for immunocharacterization and the relative number of positive cells per mm2 were cuantitated in at least ten microscope fields.

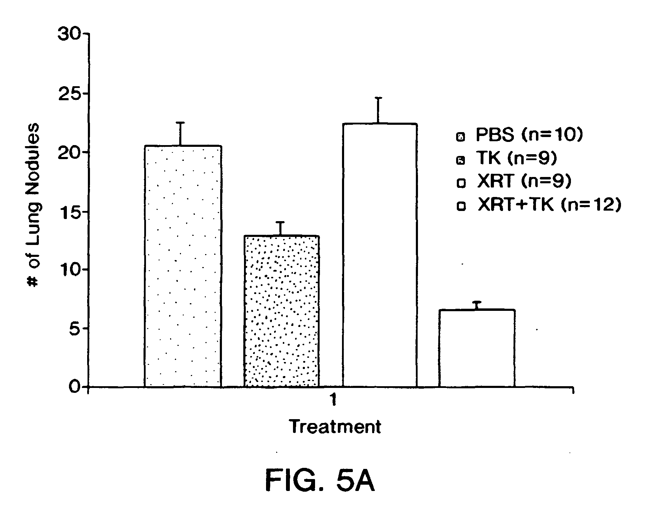

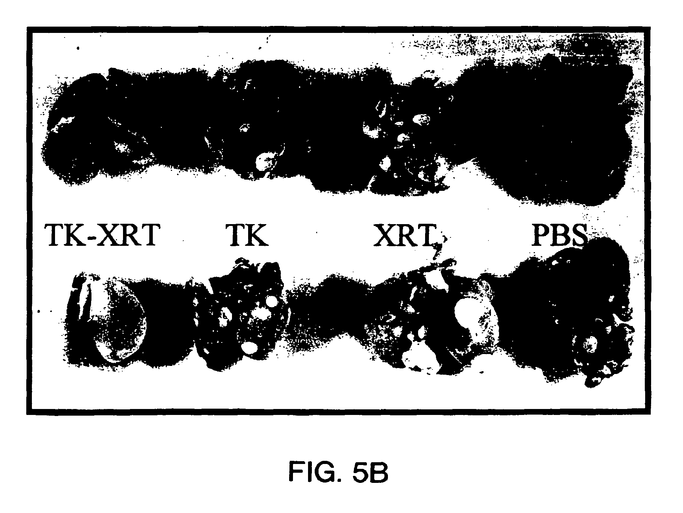

Figure 5 shows the antimetastatic effect of the combined therapy. A) bar graph of the number of lung metastasis. B) representative lungs from each group. Tumors were established by subcutaneous inoculation of 1x10⁴ and tailvein inoculation of 5x10³ of RM-1 cells. We compared the combined therapy with the two single therapy modalities and non-treated controls. AdV/RSV-tk at a dose of 3x¹⁰ AdV-tk in 20ul was injected in the subcutaneous tumor. Ganciclovir treatment was initiated 24 hours after the HSV-tk injection and a single dose of radiation of 5 Gy was given 72 hours later. The chest cavity was investigated for tumors at day 10 following the vector injection. The lungs were dissected and fixed with picric acid and the tumor nodules were counted. The HSV-tk therapy showed significantly reduced lung colonization (p<0.05), however the combination therapy resulted in further significant reduction of the number of metastasis in the lung (p<0.05).
Figure 6. shows that adenovirus encoded HSV-tk transduced murine fibroblasts and supported MHC class II dependent T cell proliferation. A) Western blot analysis of cell lysates from Ad5tk transduced DR-1 transfected L cell lysates. 30x10⁶ cells were lysed and separated on SDS-PAGE followed by transfer to nitrocellulose membranes. The membranes were probed with rabbit polyclonal antiserum raised against HSV-tk and visualized with donkey anti-rabbit HRP and ECL. Purified HSV-tk was used as a positive control. No bands were seen in Adenoviral transduction with _-galactosidase. B) DR-1⁺ and parental L cells were transduced with 1 x 10⁹ vp/ml and incubated for 24-36 hours. APCs were treated with mitomycin C and washed. APC were plated at 5x10⁴ in 96 well round bottom flasks with 2.5x10⁵ purified T cells and incubated as described in materials and methods. β-galactosidase was used to determine T cell responses to viral antigens. This served as the negative control. Adenovirus containing VSAg-7 (a well characterized viral superantigen) was used as a positive control. CPM of VSAg-7 in the presence of MHC class II molecules exceeded 80,000. In the absence of a specific presenting molecule cpm levels were less than 3000. The data are from one representative experiment done in quadruplicate. Similar results were obtained with various donors. C) DR-1, DQ and DP isotypes were used to support adenovirus transduction and subsequent T cell proliferation. APCs were transduced at 1x10⁹ vp/ml and treated as previously described. APCs were serially diluted before addition of T cells. Similar results were obtained with three individuals.
Figure 7 shows the stimulation of T cells is specific to HSV-tk and not to adenovirus or inserts. A) Ad5tk was titrated to determine the optimal concentration of adenovirus which will support T cell proliferation. At very high concentrations an obvious cytopathic effect was seen and cellular death was evident. The optimal concentration we found was 1 x 10⁹ vp/ml. Lower concentrations did however support minimal T cell proliferation. B) Wild type Ad5 was titrated in a similar manner. In spite of a broad dose curve we never observed any measurable T cell prolfieration. C) and D) T cell proliferation is specific to HSV-tk. We tested several adenovirus containing commonly used inserts for the ability to support T cell proliferation. Shown here are two representative inserts with two representative donors. These results were repeated several times with similar results.

### DETAILED DESCRIPTION OF THE INVENTION

As noted above the present invention is directed toward use of medicaments for inhibiting tumor growth and improving therapy in solid tumors and metastasis.

Briefly, the ability to ablate large numbers of tumor cells and stimulate a systemic anti-tumor effect by introduction of the Herpes thymidine kinase gene into tumor cells with a gene delivery vehicle (GDV), followed by treatment with the prodrug gancyclovir or analogous compounds, and to do so in the context of radiotherapy provides an attractive and easily usable addition to standard-of-care therapy. In addition, if the therapy is not antagonistic to, or is even collaborative with radiotherapy then clinical trials will be much simpler, physicians and patients will be more willing to use the new therapy, and additional clinical benefit to the patient will occur. However a further issue is the complication and expense of new therapies. A major cost issue for clinical therapy is the length of hospital stay and/or the number of doctor or nurse visits to the patient. Thus therapies such as gancyclovir infusion (normally for several hours every day for 14 days) normally require hospitalization or expensive home care for this period. If this is not necessary for conventional treatment, the added burden of the hospital stay and expense and inconvenience will lead to less frequent use of the new therapy. Therefore, one embodiment the present invention is the use of prodrugs that are easy to self-administer such as the orally available prodrugs valacyclovir and famcyclovir.

### GENE CONSTRUCTS

Gene constructs of various kinds are embodied in the invention. The thymidine kinase gene will normally have a promoter, a coding sequence and a polyadenylation site if it is in a DNA format. The promoters are viral or non viral and in one embodiment are designed to give tissue specific expression such as from the PSA promoter for prostate tissue; in another embodiment they are designed to give tumor specific expression from a promoter that responds to turning of the cell cycle or responds to alterations in the tumor cell that lead to expression of otherwise silent genes such as the p21 promoter in p53 mutant tumor cells; in yet another embodiment they are designed to give promiscuous expression in most tissues such as with the Rous Sarcoma Virus LTR promoter or CMV immediate early promoter. In further embodiments, tissue and/or tumor specificity is conferred by enhancer elements, locus control regions, specific RNA nuclear export or splicing arrangements, translational control elements, cell type specific protein processing activities and other cell specific biochemical processes.

In a preferred embodiment the prodrug activating gene is driven by the RSV LTR promoter.

### PRODRUG ACTIVATING GENES (PDAG) AND DRUGS

Prodrug activating genes are genes, the protein product of which encodes an activity that metabolizes an inactive drug to a pharmaceutically active form that inhibits cell proliferation, kills the cell and in some cases neighboring cells (the "bystander effect" Touraine et al. Hum Gene Ther. 9: 2385-2391 1998) or otherwise inhibits some of its functions (e.g. the ability to metastasize, the ability to secrete angiogenic promoters, the ability to inhibit the immune system and other abnormal properties of tumor cells or the cells in the tumor bed or area). The HSV TK gene is used in the present invention in conjunction with gancyclovir, acyclovir, valacyclovir or famcyclovir; as are other herpes virus TK genes with the same prodrugs.

### OTHER GENES OR FUNCTIONS ALSO CARRIED IN THE VECTOR

In a further embodiment of the present invention, the gene delivery vehicle also carries additional exogenous genes as separate coding regions, or as fusions with the thymidine kinase gene, in addition to the thymidine kinase gene. In another aspect of the invention, the thymidine kinase gene products have additional properties to that of prodrug activation, for example hyper antigenic activities. In a further embodiment of the present invention, the additional exogenous genes are other PDAG, or fusions with these such as the TK-cytosine deaminase fusion; in a yet further embodiment of the invention the additional exogenous genes encode cytokines such as interferons, α, β or γ, colony stimulating factors such as GMCSF, GCSF, MCSF; in a further embodiment the additional exogenous gene is chosen from cytokines such as IL1, IL2, IL3, IL6, IL7, IL8, IL 10, IL12, IL13, IL15, TNF; in another embodiment the additional gene is a chemokine such as rantes, Myp 1α, CXCR4 ligand, or is an immunological co-stimulatory molecule such as B7.1, B7.2, beta2microglobulin, NK stimulatory molecules, ICAM 1,2,3, HLA molecules; in a further embodiment the additional gene encodes transport molecules for the prodrug such as cell receptors or channels; in a further embodiment the additional gene encodes molecules promoting the bystander effect such as connexins, fusions with VP22 or HIV-tat that promote neighboring cell uptake of exogenous proteins, in a further embodiment the additional gene encodes antiangiogenic agents such as endostatin, angiostatin and beta interferon, procoagulant genes such as Tissue Factor or variants of these. The protein from the thymidine kinase gene can also have other anti tumor activities such as stimulating an immune response as a conventional foreign antigen or as a super antigen (see example X), that then has an antitumor effect by immune "spreading" whereby an immune response against one antigen in a cell leads to immune responses to other antigens, even autoantigens, in that cell, that would not otherwise have been seen as immunogenic.

### THE GENE DELIVERY VEHICLE

Replicating vectors of various types (see for example D. J. Jolly in Friedmann 1999, op. cit) have been proposed in part to meet the issues outlined above and in part to try to take advantage of the natural properties of the virus concerned. These are based on, for example poxviruses, adenoviruses (US5998205, US 5871726) herpes viruses (US5728379), parvoviruses, alpha viruses, retroviruses, rhabdoviruses and others.

The vector constructs of the present invention are used with adenoviral vectors including 1st (E1 deleted), 2nd (E1, E4 or E2 deleted), or 3rd ("gutless") generation adenoviral vectors. In a further embodiment of the invention, the GDV is a modified adenoviral vector capable of targeting specific cell types by means of binding to specific cell markers, the ligand for which has been engineered into the viral vector coat (Printz et al Hum. Gene Ther. 11; 191-204 2000). Gene delivery vehicles of the present invention may also be adenoviral vectors of various types including vectors with conditional expression, or mini-adeno types (Berkner, Biotechniques 6:616-627, 1988, Rosenfeld et al., Science 252:431-434, 1991, Kochanek, Hum. Gene Ther. 10:2451 1999; Steiner et al. Cancer Gene Therapy 6:456-464 1999; Chen et al. Proc Natl. Acad. Sci USA 94 1645-1650 1997; Zhang Cancer Gene Ther. 6: 113-138 1999).

In addition, viral carriers may be homologous or non-pathogenic (defective).

### USE AND SYNERGY WITH STANDARD OF CARE TREATMENTS

The present invention uses the GDV treatments in conjunction with radiotherapy. As described in the background to the invention there are four major classes of cancer therapies: surgery, radiation, chemotherapy and immune therapies. Described below is an example of types of evaluations and resultant varieties of therapies based on well characterized outcomes that occurs in determining how to treat patients with prostate cancer. Almost all cancers of any measurable occurrence are dealt with in a similar fashion and so, although the treatment decisions appear at first to be complex, they are in fact based on extensive clinical experience and can be quite easily understood as a set of treatment rules. This constitutes the "standard of care" therapy and is the treatment that most physicians will prescribe, that their colleagues will accept as normal, that insurance companies will pay for and that cannot arbitrarily be substituted by a therapy without the equivalent clinical track record. For example prostate patients at stage T2 with a Gleason score of less than 6 and PSA levels less than 10ng/ml are treated either with radiation therapy, either by external beam radiation or implantation of radio-active pellets (brachytherapy) or by surgical intervention based upon the considerations described above. Breast cancer patients are treated with external beam radiation after removal of the primary tumor and pancreatic tumor patients, who are almost all advanced disease at time of diagnosis, are treated with radiation therapy. These standard of care therapies are well known to oncologists and those skilled in the art and many can be found in textbooks, or guidelines that are routinely published in journals, by societies or other interested groups. Examples of standard of care documents are those available for specific cancers from the National Comprehensive Cancer Network, Rockledge, PA 19046 (http://www.nccn.org) published in the journal Oncology, the versions for patients available from the American Cancer Society, US National Institutes of Health Consensus Statements (http://odp.od.nih.gov/consensus/cons/cons.htm) on medical topics and reviews of specific diseases and cancer published in the New England Journal of Medicine from time to time (e.g Jay R. Harris et al., "Medical Progress: Breast Cancer", New Eng. J. Med Vol. 327:319, July 30 1992).

In a preferred embodiment of the current invention, the prodrug activating activity or the other activities that a GDV encodes are not antagonistic with radiotherapy
and in a particularly preferred embodiment the gene therapy is cooperative with the radiotherapy.

The terms collaborative, cooperative, or synergistic effect all refer to the situation where the cumulative effect of two or more agents is greater than the expected additive effect of the the agents individually. This can be determined in a number of ways known to those skilled in the art. For example Webb, J.L. (1963) in Enzyme and Metabolic Inhibitors, volume 1, Academic Press describes the original definition of these terms for biochemical reactions and A.M. Lopez et al (1999) Proc.Natl Acad Sci US 96:13023-13028 review this for more complicated situations such as mouse tumor models.
Antagonism is the opposite of synergy where the combined effects of two or more agents is less than additive. Agents could still be used together usefully, even thought they are formally antagonistic by this definition. This is also true of agents that are simply additive.

Therefore, lack of antagonism or cooperation can be ascertained by use of these therapies in mouse models in conjunction with the gene therapy. While it is well known that the extrapolation of the results of tumor treatment with single agent therapies to man is not completely predictable, determinations of synergy or otherwise between two agents should be reliable and easy to accomplish in these models. An example of such an assay for prostate tumor transduced with HSVTK followed by gancyclovir and radiation therapy is given in Example 2.

### METHODS OF ADMINISTRATION, DOSE AND FORMULATION

Within another aspect of the present invention GDV constructs carrying the thymidine kinase, are administered to a solid tumor in a patient at various doses ranging between 10⁴ and 10¹⁵ effective particles. Titres of adenoviral vectors used in the clinic typically range between 10⁸ and 10¹³ vp/ml. Patients can be injected with 0.3 to 500 ml of vector with single or repeated doses. The tumor lesion may vary from 1 to 20cm in size, for example, in the case of soft tissue sarcoma, multiple myeoloma, or head and neck squamous cell carcinoma. Patients can receive escalating doses of 0.3, 0.5 or 1.0 ml/injection for 5 days. However, since the dose may be dependent on tumor size or location, up to 500 mls of vector can be administered per day. The dose can be administered in a single injection or in multiple injections within the same tumor site over a time period. Alternatively, a dose consisting of up to 500 mls per day, can be administered over a time period of 5 days in order to establish one course. Patients can receive as many courses as necessary in order to establish a response without proving toxic. Courses can be given, for example, weekly or every other week over months or over years.

As noted above, pharmaceutical compositions are described comprising a GDV carrying the thymidine kinase vector construct, in combination with a pharmaceutically acceptable carrier or diluent (see Nyberg-Hoffman and Aguilar-Cordova, Nature Medicine, April 1999 -the contents of which are included here by reference). The composition may be prepared either as a liquid solution, or as a solid form (e.g., lyophilized) which is suspended in a solution prior to administration. In addition, the composition may be prepared with suitable carriers or diluents for surface administration, injection, oral, or rectal administration.

Pharmaceutically acceptable carriers or diluents are nontoxic to recipients at the dosages and concentrations employed. Representative examples of carriers or diluents for injectable solutions include water, isotonic saline solutions which are preferably buffered at a physiological pH or a pH for vector stability (such as phosphate-buffered saline or Tris-buffered saline), mannitol, dextrose, sucrose, glycerol, and ethanol, as well as polypeptides or proteins such as human serum albumin.

Various methods may be utilized within the context of the present invention in order to directly administer the vector construct to the tumor, including direct intralesional injection, iv administration or topical delivery. For example, within one embodiment a lesion may be located, and the vector injected once or several times in several different locations within the body of the tumor. Alternatively, arteries or blood vessels, which serve a tumor, may be identified and the vector injected into such blood vessel, in order to deliver the vector directly into the tumor. Within another embodiment, a tumor that has a necrotic center may be aspirated, and the vector injected directly into the now empty center of the tumor. Within yet another embodiment, the vector construct may be directly administered to the surface of the tumor, for example, by application of a topical pharmaceutical composition containing the vector construct, or preferably, a recombinant viral vector carrying the vector construct. Vector particles may be administered either directly (e.g., intravenously, intramuscularly, intraperitoneally, subcutaneously, orally, rectally, intraocularly, intranasally, intravesically, during surgical intervention) to the site of a tumor lesion, or the vector construct may be delivered after formulation by various physical methods such as lipofection (Felgner et al., PNAS 84:7413-7417, 1989), direct DNA injection (Fung et al., PNAS 80:353-357, 1983; Seeger et al., PNAS 81:5849-5852; Acsadi et al., Nature 352:815-818, 1991); microprojectile bombardment (Williams et al., PNAS 88:2726-2730, 1991); liposomes of several types *(see, e.g.,* Wang et al., PNAS 84:7851-7855, 1987); CaPO₄ (Dubensky et al., PNAS 81:7529-7533, 1984); DNA ligand (Wu et al., J. Biol. Chem. 264:16985-16987, 1989); administration of nucleic acids alone (WO 90/11092); or administration of DNA linked to killed adenovirus (Curiel et al., Hum. Gene Ther. 3:147-154, 1992); via polycation compounds such as polylysine, utilizing receptor specific ligands; as well as with psoralen inactivated viruses such as Sendai or Adenovirus, by electroporation or by pressure-mediated delivery. In addition vector particles or formulated construct may either be administered by direct injection to the desired site or by other clinically acceptable means such as by various forms of catheter that can be introduced into the patient with minimal discomfort, followed by injection or release of the vector in conjunction with operations made possible by the catheter, such as multiple injection, introduction of radioactive seeds, tissue disruption and other means known to those skilled in the art.

Vector particles and formulated vector constructs may be administered to a wide variety of tissue and/or cell types where lesions may exist, including for example, the brain and/or spinal cord, bone marrow, eyes, the liver, nose, throat and lung, heart and blood vessels, spleen, skin, circulation, muscles, prostate, breast, pancreas, kidney, cervix, and other organs.

### EVALUATION OF PATIENTS WITH CANCER

Every patient with any form of tumor is followed and staged in different ways depending on the tools and methods available and their accumulated track record in allowing reliable evaluation and effective disease management (for example see Medical Oncology : Basic Principles and Clinical Management of Cancer by Paul Calabresi, Philip S. Schein McGraw-Hill 1993, and Cancer, Principles and Practice of Oncology 5th edition, Vincent T. DeVita, Jr., Samuel Hellman and Steven A. Rosenberg Eds. Lippincott-Raven 1997)). For example, testicular cancer is followed with Beta HCG, AFP, and LDH, colon cancer is often followed with CEA levels, prostate cancer is followed with PSA levels, and ovarian cancer is followed with CA-125 levels. Tumors of the Head and Neck area, of the upper air and digestive tract are often followed by visual inspection as they are in an easy location to visualize. This is also the case for cervical cancer. Areas that are not easily visualized or palpated are evaluated with CT scans or MRI scans. MRI scans are extremely helpful in the evaluation of tumors of the neuraxis and the central nervous system. PET Scan is not routinely utilized to follow cancer. In the proceeding paragraphs prostate cancer is used as an example of the staging and treatment decisions of a solid tumor disease.

### PROSTATE CANCER. STAGING OF PATIENTS AND THERAPIES USED

As noted above prostate cancer affects a large number of patients with around 180,000 new diagnoses/year in the USA. The staging and treatment of patients is standardized and well defined (see e.g. J.E. Oesterling, Z.Fuks, C.T.Lee and H.I.Scher "Cancer of the Prostate" in Vincent T. DeVita Jr. et al. eds op cit 1997, Chapter 33.4and W.J. Catalona N.EngJ.Med. 331:996-1004 1994)

Prostate cancer is defined by the TNM (tumor size, nodal status, metastisis) staging system.

**Table 1 AJCC Staging System for Prostate Cancer**

| Primary Tumor | | |
|---|---|---|
| TX | | Primary tumor cannot be assessed |
| T0 | | No evidence of primary tumor |
| T 1 | | Clinical inapparent tumor not palpable or visible by imaging |
| | T1a | Tumor incidental histological finding in 5% or less of tissue resected |
| | T1b | Tumor incidental histological finding in more than 5% of tissue |
| | | Resected |
| | T1c | Tumor identified by needle biopsy (e.g. because of elevated PSA) |
| T2 | | Palpable tumor confined within prostate¹ |
| | T2a | Tumor involves one lobe |
| | T2b | Tumor involves both lobes |
| T3 | | Tumor extends through the prostatic capsule² |
| | T3a | Extra-capsular extension (unilateral or bilateral) |
| | T3b | Tumor invades seminal vesicle(s) |
| T4 | | Tumor is fixed or invades adjacent structures other than the seminal vesicles; bladder neck, external spincter, rectum, levator muscles, and/or pelvic wall |

| | | |
|---|---|---|
| ¹Tumor found in none or both lobes by needle biopsy, but not palpable or reliably visible by imaging is classified as T1c. ²Invasion into the prostatic apex or into (but not beyond) the prostatic capsule is not classified as T3, but as T2 | | |

### B. Regional Lymph Nodes (N)

| | |
|---|---|
| NX | Regional lymph nodes cannot be assessed |
| N0 | No regional lymph node metastasis |
| N1-N3 | Metastasis in regional lymph node or nodes |

Patients with documented distant nodal disease or metastatic disease are not thought to be candidates for radiation therapy. These patients would be treated with hormonal manipulation. Patients are divided into two groups depending on their T stage (see table 1, from AJCC Cancer Staging Manual, 5th edition p220, 1997, Lippincott-Raven Publishers, Philadelphia, Pennsylvania, USA), pretreatment Gleason score (pathological score based on evaluation under low power magnification a score is assigned - see Gleason D.F., "Histological grading and staging of prostatic carcinoma"; in Urologic Pathology - The Prostate M. Tannenbaum Ed. 1977, 171- 198 Lea & Febiger Philadelphia) and pretreatment prostate specific antigen (PSA) levels in the bloodstream. Good prognostic patients do not require further staging to look for metastatic disease. This group of patients has T2a disease or less, Gleason score less than or equal to 6, and a pretreatment PSA < 10 ng/ml. Patients that do not fall in this grouping require a bone scan and CT scan of the abdomen and pelvis along with a chest x-ray. Patients in the bad prognostic group receive neo-adjuvant hormonal therapy along with the radiation.

Patients in the good prognostic group have an option of surgery or radiation therapy as their primary treatment. The surgeon will decide on whether the patient is a surgical candidate base on age and risk factors (such as severe medical problems). In general a patient that is over 70 years of age will be treated with radiation therapy. The surgeon often uses the Partin tables to describe the risk of lymph node involvement, seminal vesicle involvement, or extra-capsular spread at the time of surgery.

Prognostically unfavorable patients have a high likelihood of having these pathological features at the time of proposed surgery and this would then deter the patient from undergoing surgery. Patients that undergo radiation therapy are followed closely with PSA levels. It is expected that the PSA levels in the bloodstream will show a nadir approximately 18 months after the patient has completed the radiation. The PSA level in the bloodstream should be less than 1.0 ng/ml. The patients with poor prognostic pretreatment features are given hormones for two months duration prior to being given radiation. The hormones are typically given throughout the course of the radiation therapy. At this point in time no one knows how long the patient should remain on the hormonal therapy and it is frequently stopped at the completion of radiation therapy. PSA levels should again show a nadir at approximately 18 months after the completion of the radiation unless the patient is on a protracted course of hormonal therapy. If this is the case there is a defined timeline as to when the PSA nadir should occur. PSA nadir of less than one is an indicator of disease control approximately 80% of the time.

Outcomes are quite well defined and documented for the various stages and treatments. These are measured as percent of patients with no evidence of disease at 5 and 10 years after treatment as measured by PSA levels and clinical examination. (SeeJ.E. Oesterling, Z.Fuks, C.T.Lee and H.I.Scher "Cancer of the Prostate" in Vincent T. DeVita Jr. et al. eds op cit 1997, Chapter 33.4) It is clear from these numbers that the outcomes are far from perfect and that considerable scope for improvement exists. For example somewhere around 50% (depending on the study) of T2 patients have recurrence of evidence of disease 5 years after either surgical or radiation therapies. This number becomes 70% after radiation therapy for T3/4 stage disease (surgery is not an option for these patients).

### Example 1: Adenoviral Vectors and their construction

AdV.RSV-tk is a replication-defective recombinant adenovirus carrying the HSV-tk gene under the transcriptional control of the RSV-LTR promoter. AdV-βgal is a similar vector with a β-galactosidase (β-gal) expression cassette. These vectors have been described and were prepared by standard methods (Chen et al, Proc. Nat1. Acad Sci USA 92:2577-25812 1995, Nyberg-Hoffman et al, Nature Medecine 3:506-511, 1998). Vector particle (v.p.) titer was determined by spectrophotometric absorption and infectious unit (i.u.) titer by serial dilution using end-point cytopathic effect on the 293 cell line as described (Nyberg-Hoffman et al., 1998 op.cit.). Both vectors had titers in the range of 5X10¹² vp/ml with a vp to infectious unit (IU) ratio of less than 20.

### Example 2: Animal tumor models showing cooperation of adeno TK, prodrug and radiation therapy

The mouse prostate cancer cell line RM-1 was derived from mouse prostate reconstitution (MPR) tumor tissue as described (Lu et al, 1992; Baley et al, 1995). Cells were maintained with routine media changes, expanded, and stored in liquid nitrogen at passage 8 or 9. A fresh vial of frozen RM-1 cells was used for each experiment.

### Tumor Model and Vector Injection

Immuno-competent C57BL/6 and Balb/c mice from Jackson Laboratory were used for RM-1 injections. Animals were maintained in facilities approved by the American Association for Accreditation of Laboratory Animal Care. All animal studies were conducted in accordance with the principles and procedures outlined in the National Institutes of Health's Guide for the Care and Use of Laboratory Animals. At the time of the experiments, the animals were approximately 6 weeks old and weighed around 20 gms each. Tumors were established by subcutaneous inoculation of 1 x 10⁴ of RM-1 or TM-40D (prostate and mammary, respectively) cells in 50µl. This cell dose resulted in tumors of 50-60 mm³ after 5-7 or 21-28 days respectively. The tumor implants were in the hind flank to avoid complications of scatter radiation damage to viscera. The AdV.RSV-tk dose was determined by dose escalation studies using doses from 1X10⁹ to 1X10¹¹ v.p. in 20ul per tumor followed by GCV at 20mg/kg intraperitoneally (IP) twice daily for six days. The needle was injected through an intradermal tract before entering tumor mass to minimize leakage of the vector when the needle was withdrawn. Tumor size was measured every four days in two perpendicular axis with Vernier calipers and tumor volume was calculated by the formula (a X b²)/ 2, where a is the larger and b the smaller axis of the tumor (Miller et al, 1988).

Tumor and cell injections, vector inoculations, and radiation therapy were performed under general anesthesia using Pentobarbital at a dose of 50 ug/g. Post-procedure care included maintaining the animals in a warm environment by placing them on a heated pad until recovery from anesthesia. Animals were monitored for local infection, food and fluid intake, grooming, and activity, to identify post-procedure complications. If tumor size exceeded 25 mm maximum diameter in any axis, or an animal displayed signs of compromised health such as severe loss of body weight, difficulty in breathing, physical impairment or sparse fur, the animal was euthanized.

### Radiation Therapy

Tumors of 50-60mm³ were irradiated using an orthovoltage X-Ray generator at single doses of 5, 10 and 15 Gy. Appropriate shielding of the surrounding tissues was implemented to avoid unnecessary exposure of the viscera to radiation damage. Animals were monitored for signs of radiation-induced toxicity.

For combination therapy studies, Adv/RSV-tk or AdV/RSV-β-gal (3X10¹⁰ v.p./tumor) were injected directly into the tumors as described above. Seventy-two hours after vector injection tumors were irradiated at a single dose of 5Gy. Animals and tumor sizes were monitored as described above.

### Histology and Immunology

Tissues were fixed in formalin, paraffin embedded, cut in 5-mm-thick sections and subsequently stained with hematoxylin and eosin for histological evaluation. For immuno-histochemical analysis, tumor specimens were snap frozen with liquid nitrogen in Tissue-Tec OCT compound. They were sectioned as 6-um slices on to polylysine-coated slides and fixed in cold acetone/methanol (1:1) at -20° C for 20 min. Immunohistochemical staining was performed using the avidin-biotin-peroxidase Vectastain Elite ABC kit (Vector Laboratories, Burlingame, CA). Primary rat anti-mouse CD4, and CD8, were purchased from Pharmingen, (San Diego, CA) and anti-F4/80 (anti-macrophage) from Serotec, (Oxford, UK). Dilution of the antibodies was 1:100 for anti-CD4 and anti-CD8 and 1:400 for anti-F4/80. Immunoreaction products were visualized with DAB/H₂O₂. Positively stained cells were counted in a blinded manner under a microscope by screening the cancer area. The results were recorded as the number of immunopositive cells per mm² of cancer area.

### Statistical analysis

Statistical analysis of the data with regard to the tumor growth and immunocomponents observed in the different treatment groups were performed using the Student's T-test and ANOVA (Analysis of Variance). In addition, survival curves were compared using the Wilcoxon test.

### Establishment of AdV/RSV-tk and radiation doses

To allow evaluation of synergism between HSV-tk gene and radiation therapy, establishment of effective but non-curative doses for each agent was required. To choose the vector dose for this study, established subcutaneous mouse prostate or mammary tumors were injected directly with escalating vector doses of AdV-tk followed 24 hours later by 6 days of IP GCV injection at 20mg/Kg b.i.d. AdV-tk doses of 1X10⁹, 3x10⁹, 1x10¹⁰, 3x10¹⁰ and 1x10¹¹ (v.p.) per tumor were analyzed (Fig. 1A). All treated groups showed tumor growth delay with no statistically significant difference (p>0.1) between the three highest doses on days 4, 8, and 12 after vector injection. None of the treatment groups were curative, and there was no apparent toxicity. Based on these results a dose of 3x10¹⁰ (v.p.) per tumor was chosen for combination therapy studies.

To choose a radiation dose, 5, 10 and 15 Gy single fraction doses were compared. Tumor growth delay was observed with the three doses but there was a statistically significant difference between 5 Gy and the two higher dose groups, p< 0.002 (Fig. 1B) on days 8 and 12 after the vector injection. The higher doses showed significant tumor necrosis. No significant difference in tumor growth was observed between 5 Gy and controls (p>0.2). There were no observed systemic toxicities with any of the radiation doses. Based on this data, a radiation dose of 5 Gy was chosen for combination therapy studies.

### In vivo combined therapy

Six groups were established for combination therapy studies. The first were no-treatment controls and received only PBS injections. The second were controls for non-specific effects of vector and received AdV-β-gal injections. The third and fourth were controls for the effect of single therapy modalities and received either AdV-tk + GCV or 5Gy of XRT. The fifth were controls for potential non-specific interactions between XRT and adenoviral vectors. This group received AdV-β-gal and 5 Gy XRT. The sixth group were the test animals and received AdV-tk + GCV and 5Gy of XRT. As expected, both single therapy modalities showed inhibition of tumor growth compared to controls (fig 2A). Tumor volume stabilized and the animals looked healthy throughout the 8 days after treatment as compared to the control Adv-β-gal and PBS groups. All the animals in the AdV-tk + GCV plus radiation group appeared healthy until 12 days after treatment. A statistically significant inhibition of tumor growth was observed in the combined therapy group when compared to all other treatment groups, p<0.02 (Fig. 2A). Of note is that the AdV-β-gal control group exhibited comparable tumor growth delay to that of monotherapies on day 8 although the group had significant increased tumor growth on days 4 and 12 (p ≤ 0.006). In addition, the AdV-β-gal + XRT group exhibited greater tumor growth delay on day 8 compared to the other monotherapy groups including the AdV-tk group (p=0.029) and XRT (p=0.077), a difference that was not observed on days 4 and 12 of treatment (p>0.2).

Survival studies showed no significant difference between PBS and AdV-β-gal treated animals with a survival time of 13.8 and 14.3 days respectively. Single therapies showed an improved survival compared to the control groups(p<0.02) with median survival times of 16.4, 16.8, and 18.5 days for AdV.RSV-tk, XRT and AdV-β-gal + XRT groups, respectively,. No significant difference within the three single therapy groups was observed (p>0.1). The combined therapy group, with a median survival of 22 days, showed a significant improvement compared to either the single therapy or the control groups, p<0.01.

Histological studies revealed a moderate to high generalized lymphocytic infiltration and patchy necrotic areas in the combined therapy group. A statistically significant increase in CD4+ infiltrate was seen in this group compared to the single therapy and control groups, p<0.01. CD8+ cells were also elevated in the combined treatment group but this was not significant. No significant differences were observed in the numbers of macrophage marker positive cells between the different treatment groups (data not shown).

### Example 3: Use of adenoviral vectors in conjunction with radioactive seeds

Adenoviral vectors were placed in three 500-µl aliquots in micro-centrifuge tubes containing a radioactive gold seed with at least 100% rated activity. Gold seeds have a half-life of 2.5 days. Control aliquots were placed in a location away from those with gold seeds. The aliquots were placed in a rotating platform to maintain constant mixing. Ten to 25-ul samples were taken at 15, 30 and 60 minutes and at 8, 24, 48 and 72 hours and consequently titered. The titer of the samples in contact with the radioactive gold seeds was statistically the same as that of the control aliquots. These results indicate that it is possible to co-inject adenoviral vectors with radioactive seed placement without sacrificing adenoviral vector potency.

### Example 4: Effects on metastasis with adeno TK, prodrug and radiation therapy

Tumors were established by subcutaneous inoculation of 1 x 10⁴ and tail-vein inoculation of 5x10³ of RM-1 cells. We compared the combined therapy with the two single therapy modalities and non-treated controls. AdV/RSV-tk at a dose of 3x10¹⁰AdV-tk in 20ul was injected in the subcutaneous tumor. Ganciclovir treatment was initiated 24 hours after the HSV-tk injection and a single dose of radiation of 5 Gy was given 72 hours later. The chest cavity was investigated for tumors at day 10 following the vector injection. The lungs were dissected and fixed with picric acid and the tumor nodules were counted. As shown previously (Timme TL et al., Cancer Gene Ther. Mar-Apr;5(2):74-82.(1998)) and in the attached figures, the HSV-tk therapy showed significantly reduced lung colonization (p<0.05). However, the combination therapy resulted in further significant reduction of the number of metastasis in the lung (p<0.05).

### Example 5: Hyper-antigenic effects of HSV-TK

The use of viral vectors initiates a cascade of T cell mediated responses including cytotoxic (CTL) T cell effector function and T cell dependent antibody production in response to specific vector proteins. The cellular debris and apoptosis associated with tumor killing initiates a non-specific inflammatory response. Macrophages will infiltrate the immediate vicinity, phagocytose apoptotic bodies and present antigen to cognate T cells. This dynamic milieu allows the immune system to develop a complex response to vector, tumor and possibly transgene. Immune responses may potentiate a possible anti-tumor immune response which is beneficial.

A majority of people have circulating antibodies and memory T cells specific for the common adenovirus serotypes(*). Upon clinical challenge these people mount significant responses including antibody secretion and class switching in B lymphocytes and T cell effector function. These responses play a significant role in the success of *in vivo* clinical trials and experimental models.

Secondly an immune response to specific transgenes has been documented. It is thought that these intracellular proteins are processed and enter into the conventional Major Histocompatibility Complex (MHC) class I pathway as peptide antigens and elicit cytotoxic lymphocyte (CTL) responses from cognate T cells. Some evidence exists for the development of anti-transgene antibodies that depend upon CD4 T cells and the MHC class II pathway. Therefore the administration of transgenes by viral vectors will significantly activate the host immune system by several different mechanisms. We sought to characterize the immune response to HSV-tk, the most widely used suicide gene, in an adenoviral backbone.

To our surprise, HSV-tk can support proliferation of naïve T cells only in the presence of an MHC class II allele. This observation indicates that HSV-tk may have inherent immunostimulatory properties similar to a group of well characterized proteins termed superantigens (SAgs). By definition these proteins bind to many MHC class II isoypes outside the antigen binding groove. Subsequently this bipartite interaction is further stabilized by binding to relatively invariant regions of the TCR. Polyclonal activation ofT cells bearing the appropriate element leads to robust secretion of cytokines. This interplay with immune system underlies toxicities mediated by SAgs. These proteins are elaborated by a wide range of pathogens including but not limited to gram(+) cocci and retroviruses. This conservation of function and biological activity in spite of having no structural homology between widely divergent organisms suggests that these proteins provide a selective advantage during infection of the host or life cycle of the pathogen. It is our assertion that HSV-tk is a novel viral superantigen or weak mitogen for human T lymphocytes. This observation may have profound impact on the safety and/or efficacy of HSV-tk as a therapeutic agent in gene therapy.

### In vitro stimulatory capacity of Adenovirus containing HSV-tk

Ad5Tk was used to transduce mouse L cells (MHC class II negative) and L cell transfected with human class II (DR, DP and DQ). These cells were used as antigen presenting cells (APC) to xenogeneic, allogeneic and syngeneic T lymphocytes. DR-1 (clone D.5-3.1) transfected or parental L cells were transduced with Ad5 containing _-galactosidase, HSV-tk or VSag-7 (a mouse retroviral superantigen). We tested the ability of these cells to support T cell proliferation in context of a specific presenting molecule. HSV-tk but not the insert control supported T cell proliferation. This proliferation was only evident in the presence of a human MHC class II. The specific parameter of MHC class II dependence is not characteristic of artifacts. Next we tested whether or not HSV-tk was restricted DR-1 or whether 2 other human isotypes would support T cell proliferation. DQ and DP MHC class II transfectants were used as APC in similar T cell assays. All three alleles were able to support similar levels of titratable incorporation of tritiated thymidine. The levels of T cell stimulation were of a greater magnitude than nominal antigen responses. The shapes of the dose titration curves were characterized by a moderate rise and low maximal amplitude and independent of T cell donor and transfected allele. This feature suggests that the potency reflects a feature of HSV-tk itself. The absolute ranges of individual responses are on between 15,000 cpm-60,000cpm with the mean about 25,000 cpm.

### Vector and insert controls

We showed that HSV-tk contained within an adenoviral backbone can support T cell proliferation in the presence of an unrestricted MHC class II molecule. This T cell proliferation was deemed insert dependent due to the observation that β-galactosidase and other inserts did not support the same type ofT cell proliferation. Analysis of inserts included anti-trypsin (a secreted protein), green fluorescent protein (from jellyfish) or IDS (human protein). Secondly we also tested wild type Ad5. At various concentrations wild type Ad5 could not support T cell proliferation while cells from the same donor could respond to AdTk. These series of experiments are consistent with the observation that HSV-tk specifically promotes T cell proliferation. However, it was not clear whether or not Ad5tk is transactivating an adenoviral gene product and indirectly asserting the MHC class II dependent T cell proliferation. In order to test whether or not HSV-tk retained its mitogenicity outside of an adenoviral backbone we created double stable transfectants expressing DR-1 (as assessed by flow cytometry) and HSV-tk (as assessed by sensitivity to GCV) by plasmid mediated transfection and drug selection. Three independent clones were screened for ability to support Tcell proliferation. C5, a GCV sensitive DR-1⁺ clone, consistently supported T cell proliferation above neomycin alone plasmid. C5 expressed lower levels of MHC class II when compared to DR-1 only. This observation led us to believe that HSV-tk is not merely potentiating a mixed lymphocyte reaction to MHC class II determinants which would be invariably mismatched. Secondly, when an MHC class II matched individual was used as a donor T cells proliferated in response to HSV-tk but not DR-1 alone. These observations along with the magnitude of response (in the absence of endogenous costimulation MLR responses are generally below 5,000 cpm) argue against allogeneic response. It is interesting to note that of there was a correlation between DR-1 expression and T cell proliferation between the three stably transfected clones. A response to HSV-tk was seen in the majority of the donors but this was not absolute (20 tested). Nonetheless, the ability of many donors to respond to HSV-tk in the presence of DR-1 and not the haplotype of MHC class II for which the T cell was selected is further evidence that HSV-tk is an unrestricted MHC class II dependent T cell mitogen.

### Blastogenesis of T cells upon stimulation with HSV-tk expressing DR-1⁺ fibroblasts

Flow cytometric analysis of T cells was carried out to quantitate the percentage of cells that had undergone a shift in forward scatter versus side scatter. This type of shift is consistent with various types of mitogens and superantigens. Depending on the donor the 2-3 fold increase in the total number of blasted cells compared to the allogeneic response to DR-1. The blasted gate contained cells that were assessed by propidium iodide staining to be cycling. These cells were CD3⁺ and most were CD25⁺, suggesting that cells are activated and tritiated thymidine is a true measure of DNA turnover, not cellular thymidine levels. Cells did not stain for K^{k}, an MHC class I marker on L cells. The lack of K^{k} positive cells clearly shows that the blasted cells are not fibroblast in nature. Microscopic analysis was consistent with the flow cytometric data. Visible lymphocytic aggregates were seen in solution in the presence of HSV-tk and DR-1. Some smaller clumps of cells were seen in the longer time point with DR-1 stimulation which is consistent with an allogeneic response.

### Example 6: Treatment of patients with adeno TK, valacyclovir and external beam radiation therapy

This is an example of existing clinical study designs that are in place to analyze the effects of a combination of Adtk therapy and radiation therapy on primary and metastatic disease. As an example we use the prostate model, but the approach is not limited to this disease site.

The study is comprised of three arms (see below for subject population criteria). Arm A includes low risk patients (with favorable prognostic factors), arm B has high risk patients (with unfavorable prognostic factors) and Arm C includes patients with locally advanced disease. Patients in Arms B and C receive neoadjuvant androgen ablation as part of their standard of care. Clinical response as evaluated by changes in serum PSA level and digital rectal examination as well as by histological alterations on re-biopsy such as the presence of apoptosis, necrosis, tumor proliferation and immunologic response are assessed following HSV-tk + valacyclovir treatment. Blood samples are also taken for assessment of systemic immunological response. Serum testosterone is measured. Additionally, patients are followed closely to assess nadir PSA, freedom from PSA-progression, and freedom from local and distant progression and overall survival.

### 8.0 DESCRIPTION OF PROTOCOL

### 8.1 Patient Registration

Patients may enter this study by completing the informed consent form for this protocol. Their eligibility (Appendix 2) will be confirmed by the data manager at the Department of Radiotherapy, Baylor College of Medicine. Patients will be assigned to groups by the data manager. Participation or nonparticipation in this clinical trial will not affect other therapy for which the patient may be eligible.

### 8.2 Data Collection and Monitoring

### 8.2.1 Study Monitoring

The investigators will allow study monitors and the FDA to inspect study documents (e.g. consent forms, certificate of analysis, case report forms and pertinent hospital or clinical charts).

### 8.2.2 Reporting of Adverse Reactions

Adverse Reactions (ADRs) will be reported promptly to the FDA, RAC and IRB. ADR reports are required even if only a suspicion of a drug effect is present. Previously unknown Grade 2 or 3 reactions will be reported in writing within 10 working days. Grade 4 reactions and patients' deaths while on treatment will be reported by phone within 24 hours. A written report will follow within 10 working days.

### 8.2.3 Protocol Amendment Procedures

Revisions to the protocol will be discussed by all investigators, the FDA and others as required. If the consensus is to revise the current protocol, a formal list of changes will accompany the amended protocol and these will be submitted to the FDA, IRB at Baylor College of Medicine and other pertinent committees.

### 8.2.4 Publications resulting from the Trial

Any manuscript, abstract or presentation will be available to all of the study investigators involved in this protocol. Confidentiality of subjects and of the data from the study will be maintained within legal limits.

### 8.3 Procedures (Appendix 3) and Therapeutic Agents

### 8.3.1 Delivery of HSV-tk gene

Prior to injection, all patients will receive a broad-spectrum oral antibiotic to be taken b.i.d. beginning the day prior to injection. This will be continued for 3 to 5 days. Transrectal ultrasound will be used to localize up to 4 sites of intra-prostatic injections, two at each prostatic lobe. At day 0, a total of 1-2 cc of AdV.HSV-tk adenovirus vector (the vector identical to that used in IND-6371, 6636, and 7311) (see Appendix 4) will be injected (up to 1 cc in each lateral lobe) using a 20 gauge needle. Based on the toxicity results from the phase I dose escalation study at Baylor a total of 5 x 10¹¹ vector particles per tumor will be injected. To prevent possible outflow obstruction associated with the prostatic injection, a Foley catheter may be placed following the injection. For patients in arm A, the adenoviral intra-prostatic injections will be repeated on day 14. For patients in arm B, the adenoviral intra-prostatic injections will be repeated on days 56 and 70.

### 8.3.2 Valacyclovir Administration

Valacylcovir (see Appendix 5) treatment will begin 24 hours post each virus injection at a dose of 2 gm p.o. tid and will last for 14 days. This dose has been calculated to give a similar area under the curve (auc, h-µg/ml) as 10 mg/kg of I.V. acyclovir administered every 8 hours. This is the same dose regimen as used in IND 7311, "Phase I Study of Concomitant Adenovirus-Mediated Transduction of Ovarian Cancer with HSV-tk Gene Followed by Intravenous Administration of Acyclovir and Chemotherapy with Topotecan in Patients After Optimal Debulking Surgery for Recurrent Ovarian Cancer"). Therefore, patients in arm A will receive valacyclovir on days 1 through 14 and 15 through 28. Patients in arm B will receive valacyclovir on days 1 through 14, 57 through 70 and 71 through 84.

### 8.3.3 Androgen Deprivation

For high risk patients androgen deprivation will begin concomitantly with the first adenoviral injection at day 0. Treatment will consist of one IM injection of the LHRH agonist leuprolide acetate depot (Lupron Depot, 30 mg - Appendix 5) and flutamide (Eulexin, 125 mg x 2 orally t.i.d.- Appendix 5). This is our standard androgen deprivation regimen, 30 mg of Lupron will last for four months. Flutamide is given for the first two weeks with Lupron administration.

### 8.3.4 Radiotherapy

Radiotherapy will be initiated 48 to 72 hours following the first injection in arm A and 48 to 72 hours after the second adenovirus vector injection in arms B and C. High energy photons (>10 MV) and a total dose of 70.0 Gy given in 2 Gy/fraction will be delivered to the isodose line covering the prostate in arm A and B. For arm C, 45 Gy in 1.8 Gy/fraction will be delivered to the pelvis to cover the pelvic lymphatic and the prostate will be further boosted with 26 Gy in 2 Gy/fraction.

### 9.0 SUBJECT POPULATION AND TREATMENT EVALUATION

### 9.1 Patient Eligibility

All patients must have biopsy proven adenocarcinoma of the prostate

Patients in arm A should have the following characteristics: PSA<10, Gleason's score≤6 and Clinical stage T2a

Patients in arm B should have at least one or more of the following characteristics PSA ≥10, Gleason score >6 and Clinical stage T2b-T3.

Patients in arm C should have pathological regional lymph node involvement of prostate cancer.

No prior surgical, hormonal, or radiotherapy prostate treatment.

No evidence of metastatic disease or other malignancy (except squamous or basal cell skin cancers).

Patients must have PSA within 3 months of entry.

Patients must give study specific informed consent prior to enrollment.

Patients must have adequate baseline organ function as assessed by the following laboratory values before initiating the protocol:
serum creatinine < 1.5 mg/dL
T. bilirubin < 2.5 mg/dL, ALT, AST, GGT and AP < 2 x normal
Pts > 100,000/ml³, ANC> 1500/ml³, Hgb> 10 gm/dL
Normal partial thromboplastin time (PTT) and Pro-Thrombin Time (PT)

### 9.2 Exclusion Criteria

Evidence of metastatic disease or lymph node involvement (except regional iliac lymph nodes involvement in Arm C.

Prior prostate surgery (hyperthermia, cryotherapy, etc.)

Prior pelvic radiotherapy

Prior androgen ablation hormonal therapy (except finasteride if discontinued > 3 mo. prior to enrollment)

Patients on corticosteroids or any immunosuppressive drugs.

HIV + patients

Patients with acute infections (viral, bacterial, or fungal infections requiring therapy)

Patients with cirrhosis.

### 9.3 Pretreatment Evaluation

History and physical exam including prostate diagram, height and body weight

Hematologic-CBC, PLTS, PT, PTT

Chemistries- Na, K, CL, CO₂, BUN, Cr, LDH

Liver function tests - AST, ALT, AP, GGT, total bilirubin

Urinalysis

PSA within 3 months of enrollment

Sextant biopsies of prostate - outside pathology to be reviewed at Baylor

CT pelvis will be performed in Arm B patients to rule out lymphadenopathy

In Arm B patients CXR (posterior and anterior views), and bone scan are performed. Plain film will be performed if indicated. MRI is reserved to further evaluate any suspicious areas

In Arm A patients, testosterone level will be checked.

In Arm C patients, pelvic lymph node dissection (either open or laparoscopic) is performed. pelvic lymph node metastases need to be confirmed pathologically.

### 9.4 Evaluation During Treatment (Appendix 3)

On day 0, first ADV HSV-tk injection is performed. Blood will be taken for PSA and immunological studies.

Voiding trial: A Foley catheter, if needed, may be placed just after the viral injection and will be removed within 24 hours. If the patient is unable to void, the catheter will be replaced and voiding trials will be repeated as indicated.

Physical examination will be performed weekly to monitor for acute toxicity related to treatment.

Chemistries: CBC with platelets will be monitored weekly throughout the treatment in all patients. AST, ALT, AP, GGT, total bilirubin, LDH and Cr will be monitored on weeks 2 and 4 (Arm A); week 2, 10, 12 (Arms B, C). For patients in arm B and C a repeat PSA level will be obtained at day 56, before the second viral injection and the initiation of radiotherapy. Additional blood tests will be performed as clinically indicated. For patients in Arm A, PSA will also be drawn at week 2 and at week 6.

Immunological studies (Appendix 6) will be performed on day 14 and 28 for arm A patients and on day 56, 70 and 84 for arm B and C patients.

For patients in Arm A, testosterone level will be checked at week 6.

Level of valayclovir will be checked during treatment.

Prostate biopsies: A single transrectal-ultrasound-guided prostate biopsy will be performed on day 14 for patients in arm A and on day 56 and 70 for patients in arm B and C.

### 9.5 Post-treatment evaluation

Patients will be seen 6 weeks following the completion of radiotherapy. Then follow-up will be every 3 - 4 months for the first year and every 6 months thereafter. Evaluation at each visit will include:
physical exam with prostate diagram PSA
prostate biopsies at approximately 6 weeks, 6 months, 12 months, 18 months and 24 months after the completion of radiotherapy, for the assessment of persistent or recurrent local disease

Semen samples will be sought and subjected to PCR analysis for possible viral contamination at 6 weeks (first follow-up visit) and, if positive, on every subsequent follow-up visit until it becomes negative. However, some patients will be unable to provide such samples

Immunological studies (serum) at 6 weeks

Testosterone level will be checked at six weeks for patients in Arm A.

It is understood that the performance of an individual study or test as specified in this protocol is subject to factors such as patient compliance, schedule difficulties, equipment malfunction, or the clinical judgment of the principal investigator or patient care physicians, and that a test may not be done in an individual instance without violating the protocol. However, any systematic modification of the original protocol in this regard, whether related to patient safety or not, will be submitted to the IRB. An attempt will be made to perform a complete autopsy on any patient who dies. In addition to standard pathological examination, tissues may be taken for PCR analyses for the detection of persistent recombinant adenovirus DNA.

### 9.6 Criteria for Tumor Response to Treatment

Complete response is defined as a decrease in serum PSA to < 1.0 ng/ml.

Partial response is defined as a decrease in serum PSA level by at least 50% as compared to pretreatment baseline.

Minimal response is defined as a decrease in serum PSA level by at least 25% (but less than 50%).

Stable disease is defined by one of the following: a stable serum PSA level, a minimal decrease of serum PSA level of less than 25%, or a minimal increase of the serum PSA level of no more than 20% of its initial value.

Progressive disease is defined as an increase in PSA over 20% of pretreatment baseline.

Also, results of DRE, repeat biopsies, rate of PSA decrease, time and duration to reach nadir PSA, free PSA, are assessed in the evaluation of tumor response to treatment.

### 9.7 Disease relapse

Any three successive rises in PSA will be scored as a treatment failure. The time to treatment failure will be the time interval from the date of the viral injection and initiation of androgen deprivation until the date of the first elevated PSA in the series of rising PSAs.

Local failure: Positive biopsy at two years will be scored as a local failure. Patients with increasing local disease on digital rectal exam and a rising PSA as described above will also be scored as local failures.

Distant failure: All patients with a negative DRE and a negative transrectal ultrasound-guided biopsy who have a rising PSA with or without radiographic evidence of metastatic disease will be scored as distant failures but locally controlled.

### 9.8 Toxicity and Quality of Life (QOL)

Toxicity to the HSV-tk + valacyclovir and androgen deprivation will be assessed by the Common Toxicity Criteria published by the CTEP of NCI (Appendix 7). The Radiation Therapy Oncology Group (RTOG) Acute and Late Morbidity Scorings (Appendix 8) will be used to grade toxicity from the radiotherapy. International Prostate Symptoms Score (I-PSS) and Quality of Life questionnaires (Appendix 9 & 10) will be given to patients for evaluation.

### 9.9 Treatment Cessation Criteria

a. Permanent Grade 3 toxicity or recurrent Grade 4 toxicity as specified in the CTEP or RTOG.
b. Other anticipated toxic phenomena that are not related directly to viral transduction of the prostate tumor (such as toxicity secondary to valacyclovir administration) or complications which can be explained by the natural history of malignant prostate tumors, will not be considered sufficient indication for termination of the study. Any additional occurrence of toxicity will be discussed with the Chairman of the IRB.
c. Upon request of the patient or the person with the power of attorney the patient may withdraw from the study.

### 10.0 POTENTIAL RISKS AND DISCOMFORTS

### 10.1 Potential Complication of vector injection

a. Prostatic and urinary tract infections and systemic sepsis are possible because of the transrectal route of administration, but occurs in less than 1% of patients who undergo prostate biopsy. Antibiotic therapy will be given as prophylaxis prior to and after injection of the virus into the prostate.
b. Gross hematuria following prostate biopsy occurs in <1% of patients and is usually self limited.
c. Urinary retention may result from local swelling. A Foley catheter may be placed at the time of the procedure and left in for 24 hours.

### 10.2. Potential Complications Specific to Adenovirus Vector Injection

In addition to complication resulting from an injection into the prostate, there may be complications that are specifically associated with vector treatment. No significant such complications have been observed in patients treated with the proposed dose of AdV-HSV.tk to date.

### 10.3. Potential Biohazard Exposure to Health-Care Personnel:

a. Personnel involved in vector injection: There is a low potential for biohazard exposure to the personnel involved in vector injection. The viral vector is suspended in a small volume (~1.0 ml) of buffer and is contained in a cryovial. The syringe is loaded from the vial with no vector loss. Intratumoral injection is made and the amount of leakage along the needle track is likely to be very minute. In the event of accidental spills of the vector from the vial common disinfectants can be used to inactivate the agent. All personnel are instructed on the safe use and potential biological hazards of the vector before the procedures are performed.
b. Post-surgical health care workers: There is a potential that ADV/HSV-tk virus may escape the patient via urine, feces, saliva, mucus, tears or other excretions. Studies in non-human primates indicate that the potential is low. In studies with six baboons only one plaque was detected in the serum of one baboon injected 2 days previously with the vector (see IND application 6371 or RAC Protocol 1294-098). In a semi-permissive host, cotton rats, only minimal virus was detected after intracardiac injection (Rojas et al). Exposure to shedded replication-defective virus will pose a very limited hazard, as the titers will be extremely low. There is a theoretical possibility that the vector could recombine wild-type adenovirus harbored by the patient (see above). Infection with this virus would cause a mild, self-limited infection similar to a common adenovirus infection. Health care workers will be instructed on the safe use and potential biological hazards of the virus vector before they are allowed to work with the patients. '

### 10.4. Valacyclovir (Valtrex®)

Valacyclovir caplets are for oral administration. Valacyclovir is a valine-ester of acyclovir, which is quickly converted to acyclovir by first pass intestinal and/or hepatic metabolism. Peak plasma valacyclovir concentrations are generally less than 0.5 mcg/ml at all doses.

Clinical studies comparing IV acyclovir to oral valacyclovir delivery have shown that 10 mgr/kg of IV acyclovir over 1 hour every 8 hours and 2 grams of valacyclovir every 8 hours have a similar area under the acyclovir concentration-time curve (auc, h-(g.ml), (Phase I Study of Concomitant Adenovirus-Mediated Transduction of Ovarian cancer with HSV-tk Gene Followed by Intravenous Administration of Acyclovir and Chemotherapy with Topotecan in Patients after Optimal Debulking Surgery for Recurrent Ovarian Cancer, NIH/Office of Recombinant DNA# 9801-228). Based on the above study, this protocol will use a dose of 2 grams every 8 hours for 14 days.

The most common adverse events with valacyclovir were nausea in 6-15% of patients, headache in 14-35%, vomiting in 1-6%, dizziness or abdominal pain in 2-11%, arthralgia in 1-6% and depression in 1-7%. Laboratory abnormalities such as abnormal liver function tests were observed in 1-4% of patients while anemia, leukopenia, thrombocytopenia and elevated serum creatinine occurred in 1% of patients or less. In addition, the following adverse events have been identified in clinical practice: allergic reactions (anaphylaxis, angioedema, dyspnea, pruritus, rash and urticaria), CNS symptoms (confusion, hallucinations, agitation, aggressive behavior, mania), gastrointestinal (diarrhea), renal failure, erythema multiforme, facial edema, hypertension and tachycardia.

### 10.5. Radiotherapy

Risks from the radiotherapy include possible radiation cystitis, urethritis, and proctitis during the radiotherapy. Generally these are self-limiting and improve two to three weeks following radiotherapy. Late side effects from the radiotherapy are extremely rare and include persistent diarrhea, rectal or urinary bleeding, rectal ulceration, rectal wall necrosis, and avascular necrosis of the hips. Complications requiring major surgical intervention (colostomy, hip replacement, etc.) have been reported to occur in less than 1% of the cases.

### 10.6. Androgen Deprivation

### 10.6.1 Flutamide

The most frequently reported (greater than 5%) adverse experiences during treatment with Eulexin Capsules in combination with an LHRH agonist are: hot flashes, 61%; loss of libido, 36%; impotence, 33%; diarrhea, 12%; nausea/vomiting, 11%; gynecomastia, 9%; other, 7%; and other GI, 6%. Since transaminase abnormalities, cholestatic jaundice, hepatic necrosis, and hepatic encephalopathy have been reported with the use of flutamide, periodic liver function tests should be considered. Appropriate laboratory testing should be done at the first symptom/sign of liver dysfunction (e.g., pruritus, dark urine, persistent anorexia, jaundice, right upper quadrant tenderness or unexplained "flu-like" symptoms). If the patient has jaundice or laboratory evidence of liver injury, in the absence of biopsy- confirmed liver metastases, Eulexin therapy should be discontinued or the dosage reduced. The hepatic injury is usually reversible after discontinuation of therapy, and in some patients, after dosage reduction. However, there have been reports of death following severe hepatic injury associated with the use of flutamide.

### 10.6.2 Leuprolide Acetate.

In a clinical trial of Lupron Depot (Leuprolide acetate for depot suspension) the following adverse reactions were reported to have a possible or probable relationship to the drug as ascribed by the treating physician: edema, 12.5%; nausea/vomiting, 5.4%; decreased testicular size, 5.4%; hot flashes/sweats, 58.9%; impotence, 5.4%; general pain, 7.1%; dyspnea, 5.4% and asthenia, 5.4%. Elevation of the following laboratory studies were noted: SGOT (> 2 x normal), 5.4%; LDH (> 2 x normal), 19.6%; and alkaline phosphatase (>1.5 x normal), 5.4%.

### 11.0 COSTS

Patients, through insurance or other means, will be responsible for all standards of care drugs and procedures. There will be no patient charge for the vector, vector delivery or any other protocol-specific drug or procedure.

### 12.0 CONSENT PROCEDURE

All patients must sign an approved informed consent form in order to be eligible for this study (Appendix 11 for Low Risk, Arm A; Appendix 12 for High Risk, Arm B; Appendix 13 for D1, Arm C).

### 13.0 CONFIDENTIALITY

Confidentiality of patients' records will be maintained within legal limits. The data from this study may be published, however no patients will be named or identified.

### 14.0 POTENTIAL BENEFITS

The present study may have no benefit to the study subjects. However, pre-clinical studies show significant cell killing in human prostate cancer tumor models. Another Phase I study in humans demonstrated minimal toxicity using the ADV delivery of HSV-tk followed by GCV in recurrent prostate cancer. In prostate cancer with a large volume of local disease, radiation therapy results in less than optimal local control. Therefore in combining two local treatments with nonoverlapping toxicities, local control may be improved with little additional toxicity. Improvement in local control in patients with clinically localized prostate cancer may translate into improved disease free survival.

### 15.0 RISK-BENEFIT RATIO

A Phase 1-II study with AdV/HSV-tk gene therapy alone showed no lasting toxicity and minimal discomfort to the patients. The group of patients in this study would normally be receiving androgen ablation and/or radiotherapy as standard of care, therefore this portion of the treatment will carry no additional risk. Given the possible clinical benefit of improvement in local control, the risk-benefit ratio appears to be favorable.

THIS PROTOCOL CONFORMS WITH THE OSHA/HHS GUIDELINES FOR HIV/HBV OCCUPATIONAL SAFETY.

### Appendix 2

### GENE THERAPY ELIGIBILITY FORM

| Patient Name | | Sex | |
|---|---|---|---|
| Chart Number | | Race | |
| Hospital # | | SSN | |
| Referring MD | | Zip Code | |
| Referring Institution | | Method of Payment | |
| Telephone # | | Injection Date | |
| DOB | | Tx Assignment | |

(Y/N) 1. Biopsy proven adenocarcinoma of the prostate
2. Arm A = PSA <10, Gleason ?6, Clinical Stage T1 - T2a Arm B = one or more of the following : PSA ≥ 10, Gleason score >6 and clinical stage T2b-T3
   Arm C = pathological regional lymph node involvement of prostate cancer
(N) 3. Prior surgical, hormonal, or radiation prostate treatments
(N) 4. Evidence of metastatic disease or other malignancy (exceptions: pelvic lymph node positive, squamous or basal cell skin cancer)
(Y) 5. PSA performed within 3 months of entry; Date:
(Y) 6. Signed a Study specific informed consent form
(Y) 7. Adequate baseline organ function as assessed by the following lab values:
   Serum creatinine < 1.5mg%
   T. bilirubin < 2.5 mg/dL, ALT, AST, GGT and AP < 2 x normal
   Pts > 100,000/mm³, ANC > 1500/ml³, Hgb > 10gm/dL
   Normal partial thromboplastin time (PTT) and Pro-Thrombin Time (PT)
(N) 8. Is the patient on corticosteroids or any immunosuppressive drugs?
(N) 9. Does the patient have any acute infections (viral, bacterial, or fungal infections requiring therapy?
(N) 10. Does the patient have cirrhosis?
(Y) 11. Is the patient HIV negative? How did the patient learn about the patient learn about gene therapy?
Completed By:
Date:

### Appendix 3 Phase I-II Study Evaluating HSV-tk + Valacyclovir Gene Therapy in Combination with Radiotherapy for Prostate Cancer TREATMENT FLOW SHEET (refer to Section 8.3 and 9.4 for details) (A: Arm A B: Arm B C: Arm C)

| Week | 0 | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|---|
| HSV-tk 1-2cc inj. | Day 0 A,B,C | | | Day 14 A | | | | | Day 56 B,C | | | Day 70 B,C | | | | | |
| Valacyclovir 2gm tid x 14 | | A, B, C | A, B, C | A | A | | | | | B, C | B, C | B, C | B, C | | | | |
| Lupron 30mg im | Day 0 B, C | | | | | | | | | | | | | | | | |
| Eulexin 125mg x 2 tid | Day 0 B, C | → | | | | | | | | | | | | | | | |
| XRT | | A | → | | | | | | | B, C → | | | | | | | |
| Biopsy | | | | Day 14 A | | | | | Day 56 B, C | | | Day 70 B, C | | | | | |
| PSA | Day 0 A, B, C | | A | | | A | | | Day 56 B, C | | | | | | | | |
| Immunologic Studies | Day 0 A, B, C | | | Day 14 A | | | | | Day 56 B,C | | | Day 70 B,C | | | | | |
| Testosterone level | Day 0 A | | | | | A | | | | | | | | | | | |
| Chemistries | Day 0 | | A, B, C | | A | | | | | | B,C | | B, C | | | | |
| CBC | Both A and B, CBC with platelets/every week | | | | | | | | | | | | | | | | |
| Urine | Daily injection for 7 days | | | | | | | | | | | | | | | | |

| | | | | | | | | | | | | | | | | | |
|---|---|---|---|---|---|---|---|---|---|---|---|---|---|---|---|---|---|
| Post-treatment evaluation: Note: 1. Tylenol #3 ii p.o. and Valium 5 mg p.o. 1 hour prior to injection a. Physical examination with prostate diagram 2. Cipro 500 mg p.o. b.i.d. from 3 days, starting the day before injection b. PSA 3. Chemistries include: AST, ALT, AP, GGT, LDH. Total Bili, Cr c. Prostate biopsies: 6 weeks, 6 months, 12 months, 18 months, and 24 months d. Semen samples: from 6 weeks to 6 months or to negative. (PCR analysis for viral contamination) e. Testosterone level at 6 weeks for Arm A f. Immunological studies (serum) at 6 weeks | | | | | | | | | | | | | | | | | |

### Example 7. Treatment of patients with adeno TK, valacyclovir and brachytherapy

This is an example of a clinical study design to analyze the effects of a combination of Adtk therapy and radiation therapy applied by radioactive seeds. As an example we use the prostate model, since the protocol is similar to the one described above and hence only a portion is included here as an example, but the approach is not limited to this disease site and is currently being pursued for other sites including but not limited to pancreatic cancer.

### 8.0 DESCRIPTION OF PROTOCOL

### 8.1 Patient Registration

Patients may enter this study by completing the informed consent form for this protocol. Their eligibility will be confirmed by the data manager at the Department of Radiotherapy, Baylor College of Medicine. Patients will be assigned to groups by the data manager. Participation or nonparticipation in this clinical trial will not affect other therapy for which the patient may be eligible.

### 8.2 Data Collection and Monitoring

### 8.2.1 Study Monitoring

The investigators will allow study monitors and the FDA to inspect study documents (e.g. consent forms, certificate of analysis, case report forms and pertinent hospital or clinical charts).

### 8.2.2 Reporting of Adverse Reactions

Adverse Reactions (ADRs) will be reported promptly to the FDA, RAC and IRB. ADR reports are required even if only a suspicion of a drug effect is present. Previously unknown Grade 2 or 3 reactions will be reported in writing within 10 working days. Grade 4 reactions and patients' deaths while on treatment will be reported by phone within 24 hours. A written report will follow within 10 working days.

### 8.2.3 Protocol Amendment Procedures

Revisions to the protocol will be discussed by all investigators, the FDA and others as required. If the consensus is to revise the current protocol, a formal list of changes will accompany the amended protocol and these will be submitted to the FDA, IRB at Baylor College of Medicine and other pertinent committees.

### 8.2.4 Publications resulting from the Trial

Any manuscript, abstract or presentation will be available to all of the study investigators involved in this protocol. Confidentiality of subjects and of the data from the study will be maintained within legal limits.

### 8.3 Procedures and Therapeutic Agents

### 8.3.1 Delivery of HSV-tk gene

Prior to injection, all patients will receive a broad-spectrum oral antibiotic the evening before and the morning of the injection. This will be continued for 5 days. Transrectal ultrasound will be used to localize up to 4 sites of intra-prostatic injections, two at each prostatic lobe. At day 0, a total of 1-2 cc of AdV.HSV-tk adenovirus vector (the vector identical to that used in IND-6371, 6636, and 7311) (see Appendix 1) will be injected (up to 1 cc in each lateral lobe) using a 20 gauge needle. Based on the toxicity results from the phase I dose escalation study at Baylor a total of 5x10¹¹ vector particles per tumor will be injected,. To prevent possible outflow_obstruction associated with the prostatic injection, a Foley catheter may be placed following the injection. For patients in arm A, the adenoviral intra-prostatic injections will be repeated within 2 - 3 weeks. For patients in arm B, the adenoviral intra-prostatic injections will be repeated on days 54 and 70.

### 8.3.2 Valacyclovir Administration

Valacylcovir (see Appendix 2) treatment will begin 24 hours post each virus injection at a dose of 2 gm p.o. tid and will last for 14 days. This dose has been calculated to give a similar area under the curve (auc, h-µg/ml) as 10 mg/kg of I.V. acyclovir administered every 8 hours. This is the same dose regimen as used in IND 7311, "Phase I Study of Concomitant Adenovirus-Mediated Transduction of Ovarian Cancer with HSV-tk Gene Followed by Intravenous Administration of Acyclovir and Chemotherapy with Topotecan in Patients After Optimal Debulking Surgery for Recurrent Ovarian Cancer"). Therefore, patients in arm A will receive valacyclovir on days I through 14 and 15 through 29. Patients in arm B will receive valacyclovir on days 1 through 14, 56 through 69 and 70 through 84.

### 8.3.3 Androgen Deprivation

For high risk patients androgen deprivation will begin concomitantly with the first adenoviral injection at day 0. Treatment will consist of monthly injections of the LHRH agonist leuprolide acetate depot (Lupron Depot, 30 mg - Appendix 2) and flutamide (Eulexin, 250 mg po tid - Appendix 2). This is our standard androgen deprivation regimen and will continue for a total of 4 months.

### 8.3.4 Radiotherapy

For Arm A: Radioactive gold seeds implant is performed (under transretal ultrasound guidance and fluorouscopic guidance.)at the same time of first intra-prostatic injection of an HSV-tk adenoviral vector on day 0. External beam radiotherapy (IMRT) will be initiated within 2 - 3 weeks following radioactive gold seed implant (the first treatment of external beam radiotherapy is delivered within 72 hours after the second adenoviral intra-prostatic injection).

For Arm B: RGSI is performed at the same time of second intra-prostatic injection of Adv-HSV-tk adenoviral vector on day 54. External beam radiotherapy (IMRT) will be initiated within 2 - 3 weeks following radioactive gold seed implant (the first treatment of external beam radiotherapy is delivered within 72 hours after the third adnoviral intra-prostatic injection).

Radioactive gold seed implant is delivered by implanting aproximately 40 seeds transperineally into the prostate gland uner transrectal ultrasound guidance. Each radioactive gold seed is approximately at 2 mCi, thus a total of approximately 80 mCi, usually delivering 20 - 30 Gy.

High energy photons (> 10 MV) and a total dose of 50 Gy given in 2 Gy/fraction will be delivered to the isodose line covering the prostate. Area of cold spots will be compensated with intensity modulated radiotherapy.

### 9.0 SUBJECT POPULATION AND TREATMENT EVALUATION

### 9.1 Patient Eligibility

All patients must have biopsy proven adenocarcinoma of the prostate

Patients in arm A should have the following characteristics: PSA<10, Gleason's score≤6 and Clinical stage T2a or below

Patients in arm B should have at least one or more of the following characteristics PSA≥10, Gleason score >6 and Clinical stage T2b-T3.

No prior surgical, hormonal, or radiotherapy prostate treatment.

No evidence of metastatic disease or other malignancy (except squamous or basal cell skin cancers.

Patients must have PSA within 3 months of entry.

Patients must give study specific informed consent prior to enrollment.

Patients must have adequate baseline organ function as assessed by the following laboratory values before initiating the protocol:
serum creatinine < 1.5 mg%
T. bilirubin < 2.5 mg%, ALT and AST < 2x normal
Pts > 100,000/mm 3 , ANC> 1500/mm³, Hgb> 10gm%
Normal partial thromboplastin time (PTT) and Pro-Thrombin Time (PT)

### 9.2 Exclusion Criteria

Evidence of metastatic disease or lymph node involvement.

Prior prostate surgery (hyperthermia, cryotherapy, etc.)

Prior pelvic radiotherapy

Prior androgen ablation hormonal therapy (except finasteride if discontinued > 3 mo prior to enrollment)

Patients on corticosteroids or any immunosuppressive drugs.

HIV + patients

Patients with acute infections (viral, bacterial, or fungal infections requiring therapy)

Patients with cirrhosis.

### 9.3 Pretreatment Evaluation

History and physical exam including prostate diagram, height and body weight

Hematologic-CBC, PLTS, PT, PTT

Chemistries-Na, K, CL, CO2, Cr, AST, ALT, LDH, Bilirubin, Ca Urinalysis

PSA within 3 months of enrollment

CXR (posteroanterior and lateral views)

Bone scan with MRI reserved to evaluate any suspicious areas

Sextant biopsies of prostate

Immunologic studies: Presence of serum neutralizing antibodies to wild type adenovirus

CT pelvis will be performed in high-risk patients to rule out pelvic adenopathy

### 9.4 Evaluation During Treatment

Physical examination. Examination will be performed at each patient visit. Patients will be evaluated monthly to receive LHRH agonist injection with regard to their tolerance to androgen withdrawal and drug-related toxicities During radiotherapy the patients will be monitored weekly for acute toxicity.

Voiding trial: A Foley catheter, if needed, may be placed just after the viral injection and will be removed within 24 hours. If the patient is unable to void, the catheter will be replaced and voiding trials will be repeated as indicated.

Chemistries: Serum creatinine, liver function tests, CBC, coagulation studies and blood chemistries will be monitored weekly throughout the treatment in all patients. For patients in arm B a repeat PSA level will be obtained at day 56, before the second viral injection and the initiation of radiotherapy. Additional blood tests will be performed as clinically indicated.

Immunological studies: Titer of serum antibodies to adenovirus on day 15 for arm A patients and on days 56 and 71 for arm B patients.

Prostate biopsies: Transrectal ultrasound-guided prostate biopsies will be repeated on the second intraprostatic injection of HSV-tk gene for patients in arm A and on the second and third injectioni of HSV-tk gene for patients in arm B.

Cystoscopic examination during radioactive gold seed implant for evaluation of seeds within bladder and for washing of virus in bladder.

### 9.5 Post-treatment evaluation

Patients will be seen 6 weeks following the completion of radiotherapy.

Then follow-up will be every 3 months for the first year and every 6 months thereafter. Evaluation at each visit will include:
physical exam with prostate diagram
PSA
prostate biopsies at 6 weeks and 6, 12, 18 and 24 months after the completion of radiotherapy, for the assessment of persistent or recurrent local disease

Semen samples will be sought and subjected to PCR analysis for possible viral contamination at 6 months (first follow-up visit) and ,if positive, on every subsequent follow-up visit until it becomes negative. However, some patients will be unable to provide such samples

It is understood that the performance of an individual study or test as specified in this protocol is subject to factors such as patient compliance, schedule difficulties, equipment malfunction, or the clinical judgment of the principal investigator or patient care physicians, and that a test may not be done in an individual instance without violating the protocol. However, any systematic modification of the original protocol in this regard, whether related to patient safety or not, will be submitted to the IRB. An attempt will be made to perform a complete autopsy on any patient who dies. In addition to standard pathological examination, tissues may be taken for PCR analyses for the detection of persistent recombinant adenovirus DNA

### 9.6 Criteria for Tumor Response to Treatment

Complete response is defined as a decrease in serum PSA to within normal limits (≤ 1.0 ng/ml).

Partial response is defined as a decrease in serum PSA level by at least 50% as compared to pretreatment baseline.

Minimal response is defined as a decrease in serum PSA level by at least 25% (but less than 50%).

Stable Disease is defined by one of the following: a stable serum PSA level, a minimal decrease of serum PSA level of less than 25%, or a minimal increase of the serum PSA level of no more than 20% of its initial value.

Progressive disease is defined as an increase in PSA over 20% of pretreatment baseline.

Also the results of DRE, repeat biopsies, rate of PSA decrease, time and duration of nadir PSA, free PSA, PAP, and alkaline phosphatase may be assessed in the evaluation of tumor response to treatment.

### 9.7 Disease relapse

Any two successive rises in PSA will be scored as a treatment failure. The time to treatment failure will be the time interval from the date of the viral injection and initiation of androgen deprivation until the date of the first elevated PSA in the series of rising PSAs.

Local failure: Positive biopsy at two years will be scored as a local failure. Patients with increasing local disease on digital rectal exam and a rising PSA as described above will also be scored as local failures.

Distant failure: All patients with a negative DRE and a negative transrectal ultrasound-guided biopsy who have a rising PSA with or without radiographic evidence of metastatic disease will be scored as distant failures but locally controlled

### 9.8 Toxicity:

Toxicity to the HSV-tk + valacyclovir and androgen deprivation will be assessed by the Common Toxicity Criteria published by the CTEP of NCI (Appendix 3). The Radiation Therapy Oncology Group (RTOG) Acute and Late Morbidity Scorings (Appendix 4) will be used to grade toxicity from the radiotherapy.

### 9.9 Treatment Cessation Criteria

a. Permanent Grade 3 toxicity or recurrent Grade 4 toxicity as specified in the CTEP or RTOG.
b. Other anticipated toxic phenomena that are not related directly to viral transduction of the prostate tumor (such as toxicity secondary to valacyclovir administration) or complications which can be explained by the natural history of malignant prostate tumors, will not be considered sufficient indication for termination of the study. Any additional occurrence of toxicity will be discussed with the Chairman of the IRB.
c. Upon request of the patient or the person with the power of attorney the patient may withdraw from the study.

## Claims

1. The use of a replication deficient adenoviral gene delivery vector for the preparation of a medicament for use in human cancer patients to decrease, or retard an increase in, localised or metastatic tumour burden, wherein the vector is to be administered to a tumour, the vector comprises a nucleotide sequence encoding thymidine kinase, and the human cancer patients are to receive gancyclovir or an analogous prodrug following administration of the vector;
**characterised in that**:
the gene delivery vector and the prodrug are to be administered in combination with radiotherapy in the cancer patient in order to induce a systemic anti-tumour immune response.

2. The use according to claim 1, wherein the vector further comprises an additional prodrug activating gene, an immunostimulatory gene, a xenoantigen, a hyperantigen or a superantigen to the tumour.

3. The use according to claim 1 or 2, wherein the prodrug is valcyclovir, famcyclovir or any other orally available analogue.

4. The use according to claim 1, 2 or 3, wherein the vector carries additional exogenous genes which are interferons, colony stimulating factors, interleukins, chemokines or costimulatory molecules.

5. The use according to any preceding claim, wherein the tumour to be targeted is a tumour of the prostate, head and neck, cervix, brain, bladder, rectum, pancreas, colon, lung or breast.

6. The use according to claim 5, wherein the tumour is a tumour of the prostrate.

7. The use according to any preceding claim, wherein the vector is formulated to be administered at a dose of 10⁹ to 10¹⁵ effective particles,

8. The use according to any preceding claim, wherein the vector is formulated to be administered at a dose of 10⁹ to 10¹³ effective particles.

9. The use according to any preceding claim, wherein the vector is formulated to be administered at a dose of 10⁹ to 10¹¹ effective particles.

## Patentansprüche

1. Verwendung eines replikationsdefizienten adenoviralen Gen-Delivery-Vektors für die Herstellung eines Medikaments zur Anwendung bei menschlichen Krebspatienten zur Verringerung oder Verzögerung der Zunahme einer lokalen oder metastasierenden Tumormasse, wobei der Vektor einem Tumor verabreicht wird, und der Vektor eine Nukleotidsequenz enthält, die für Thymidinkinase kodiert, und der menschliche Krebspatient Gancyclovir oder ein analoges Prodrug im Anschluss an die Verabreichung des Vektors erhält,
**dass** der Gen-Delivery-Vektor und das Produg dem Krebspatienten in Verbindung mit einer Strahlentherapie verabreicht werden, um eine systematische Antitumorimmunantwort zu induzieren.

2. Verwendung nach Anspruch 1, wobei der Vektor zudem ein zusätzliches Prodrug aktivierendes Gen, ein imrnunstimmulierendes Gen, ein Xenoantigen, ein Hyperantigen oder ein Superantigen für den Tumor aufweist.

3. Verwendung nach Anspruch 1 oder 2, wobei das Prodrug Valcyclovir, Famcyclovir oder ein beliebiges anderes oral verfügbares Analog ist.

4. Verwendung nach Anspruch 1, 2 oder 3 wobei der Vektor zusätzlich Interferone, kolonie-stimulierende Faktoren, Interleukine, Chemokine oder kostimulierende Moleküle als exogene Gene aufweist.

5. Verwendung nach einem der vorhergehenden Ansprüche, wobei der Zieltumor ein Prostatatumor, ein Hals- und Nackentumor, ein Gebärmutterhalstumor, ein Hirntumor, Blasentumor, Rektaltumor, Bauchspeicheldrüsentumor, Darmtumor, Lungen- oder Brusttumor ist.

6. Verwendung nach Anspruch 5, wobei der Tumor ein Prostatatumor ist.

7. Verwendung nach einem der vorhergehenden Ansprüche, wobei der Vektor so formuliert ist, um in einer Dosis von 10⁹ bis 10¹⁵ effektiven Partikeln verabreicht zu werden.

8. Verwendung nach einem der vorhergehenden Ansprüche, wobei der Vektor so formuliert ist, um in einer Dosis von 10⁹ bis 10¹³ effektiven Partikeln verabreicht zu werden.

9. Verwendung nach einem der vorhergehenden Ansprüche, wobei der Vektor so formuliert ist, um in einer Dosis von 10⁹ bis 10¹¹ effektiven Partikeln verabreicht zu werden.

## Revendications

1. Utilisation d'un vecteur de délivrance de gène adénoviral déficient de réplication pour la préparation d'un médicament à utiliser chez des patients humains atteints de cancer pour réduire ou retarder la croissance d'une charge tumorale localisée ou métastatique, dans laquelle le vecteur doit être administré à la tumeur, le vecteur comprend une séquence nucléotidique codant la thymidine kinase, et les patients humains atteints de cancer doivent recevoir du gancyclovir ou un promédicament analogue après l'administration du vecteur ;
**caractérisée en ce que** :
le vecteur de délivrance de gène et le promédicament doivent être administrés en combinaison avec une radiothérapie au patient atteint de cancer afin d'induire une réponse immunitaire antitumorale systémique.

2. Utilisation selon la revendication 1, dans laquelle le vecteur comprend en outre un gène d'activation de promédicament supplémentaire, un gène immunostimulatoire, un xénoantigène, un hyperantigène ou un superantigène dirigé vers la tumeur.

3. Utilisation selon la revendication 1 ou 2, dans laquelle le promédicament est le valcyclovir, le famcyclovir ou tout autre promédicament analogue disponible pour une administration par voie orale.

4. Utilisation selon la revendication 1, 2 ou 3, dans laquelle le vecteur véhicule des gènes exogènes supplémentaires qui sont des interférons, des facteurs de stimulation des colonies, des interleukines, des chimiokines ou des molécules costimulatoires.

5. Utilisation selon l'une quelconque des revendications précédentes, dans laquelle la tumeur à cibler est une tumeur de la prostate, de la tête et du cou, du col de l'utérus, du cerveau, de la vessie, du rectum, du pancréas, du côlon, du poumon ou du sein.

6. Utilisation selon la revendication 5, dans laquelle la tumeur est une tumeur de la prostate.

7. Utilisation selon l'une quelconque des revendications précédentes, dans laquelle le vecteur est formulé pour être administré à une dose comprise entre 10⁹ et 10¹⁵ particules effectives.

8. Utilisation selon l'une quelconque des revendications précédentes, dans laquelle le vecteur est formulé pour être administré à une dose comprise entre 10⁹ et 10¹³ particules effectives.

9. Utilisation selon l'une quelconque des revendications précédentes, dans laquelle le vecteur est formulé pour être administré à une dose comprise entre 10⁹ et 10¹¹ particules effectives.
